# EUROPEAN PATENT SPECIFICATION

(11) **EP 2 357 227 B1**
(45) Date of publication and mention of the grant of the patent: **15.07.2015**
(21) Application number: 10153355.2
(22) Date of filing: 11.02.2010
(51) Int. Cl.: C12N 9/42

(54) **Optimized cellulase enzymes**
Optimierte Zellulaseenzyme
Enzymes de cellulase optimisées

(43) Date of publication of application: 17.08.2011
(73) Proprietor: Süd-Chemie IP GmbH & Co. KG, 80333 München (DE)
(72) Inventor: Reisinger, Christoph, Dr., 81477 München (DE); Brück, Thomas, Dr., 82067 Ebenhausen (DE); Koltermann, André, Dr., 82057 Icking (DE); Gerlach, Jochen, Dr., 80687 München (DE); Unterstrasser, Isabel, 83253 Rimsting (DE); Röcher, Lutz, 81541 München (DE); Rarbach, Markus, Dr., 81369 München (DE); Claren, Jörg, Dr., 81476 München (DE); Kohl, Andreas, Dr., 81369 München (DE); Pieck, Jan, Carsten, Dr., 80689 München (DE); Schlosser, Dominik, 81369 München (DE)
(74) Representative: Graser, Konstanze

(56) References cited:
- WO-A1-2006/117432
- WO-A1-2009/139839
- WO-A1-2010/005553
- WO-A2-2009/138877
- DATABASE UniProt [Online] 1 February 2005 (2005-02-01), "SubName: Full=Cellobiohydrolase;" XP002593511 retrieved from EBI accession no. UNIPROT:Q5MBA7 Database accession no. Q5MBA7
- DATABASE PDB [Online] 24 February 2009 (2009-02-24), Grassnick et al.: "3-DIMENSIONAL STRUCTURE OF NATIVE CEL7A FROM TALAROMYCES EMERSONII" XP002593512 retrieved from EBI accession no. PDB:1Q9H Database accession no. 1Q9H
- GRASSICK ALICE ET AL: "Three-dimensional structure of a thermostable native cellobiohydrolase, CBH IB, and molecular characterization of the cel7 gene from the filamentous fungus, Talaromyces emersonii", EUROPEAN JOURNAL OF BIOCHEMISTRY, GB, vol. 271, no. 22, 1 November 2004 (2004-11-01), pages 4495-4506, XP002547329, ISSN: 0014-2956, DOI: 10.1111/J.1432-1033.2004.04409.X

## Description

### Field of invention

The invention discloses cellulase enzymes with optimized properties for processing of cellulose- and lignocellulose-containing substrates. In particular, cellobiohydrolase enzymes with preferred characteristics are disclosed. The present disclosure provides fusion, insertion, deletion and/or substitution variants of such enzymes. Enzyme variants have enhanced thermostability, proteolytic stability, specific activity and/or stability at extreme pH. Nucleic acid molecules encoding said enzymes, a composition comprising said enzymes, a method for preparation, and the use for cellulose processing and/or for the production of biofuels are disclosed.

### Background of the invention

The development of production processes based on renewable resources is highly desired, for example for the generation of ethanol from cellulosic and lignocellulosic materials.

Cellulose material in pure form or in combination with hemicellulose and/or lignin is a valuable and readily available raw material for the production of chemicals and fuels. A key step in processing cellulose and lignocellulose is the hydrolysis of the beta-1,4-linked glucose polymer cellulose and the subsequent release of glucose monomers and short glucose oligomers such as cellobiose, cellotriose, etc. Enzymes that catalyze this reaction are found in various organisms, especially filamentous fungi and bacteria, that are capable of degrading and hydrolysing cellulose.

Continuous processes for converting solid lignocellulosic biomass into combustible fuel products are known. Treatment to make cellulosic substrates more susceptible to enzymatic degradation comprises milling, chemical processing and/or hydrothermal processing. Examples are wet oxidation and/or steam explosion. Such treatments increase the accessibility of cellulose fibers and separate them from hemicellulose and lignin. required for the degradation of cellulose polymers. Among these cellobiohydrolase (CBH) enzymes, and more specifically cellobiohydrolase I (CBHI) enzymes, play a key role in the hydrolysis step as they provide the most processive enzymatic activity. CBHI enzymes catalyze the progressive hydrolytic release of cellobiose from the reducing end of the cellulose polymers. (Lynd LR, Weimer PJ, van Zyl WH, Pretorius IS. Microbial cellulose utilization: fundamentals and biotechnology. Microbiol Mol Biol Rev. 2002 Sep;66(3):506-77).

Hydrolyzed cellulosic materials contain several valuable carbohydrate molecules which can be isolated from the mixtures. Sugar containing hydrolysates of cellulosic materials can be used for microbial production of a variety of fine chemicals or biopolymers, such as organic acids, ethanol or higher alcohols (also diols or polyols) or polyhydroxyalkanoates (PHAs). One of the major uses of the sugar hydrolysates is in the production of biofuels.

**Kurabi et al. (2005)** describes preparations of cellulases from Trichoderma reesei and other fungi, such as Penicillium sp. The performance has been analysed on steam-exploded and ethanol organosolv-pretreated Douglas-fir. Better performance of enzyme mixtures appears to be a result of improved properties of single component enzymes as well as the effect of each compound in the mixture, especially the presence of beta-glucosidase. (Kurabi A, Berlin A, Gilkes N, Kilburn D, Bura R, Robinson J, Markov A, Skomarovsky A, Gusakov A, Okunev O, Sinitsyn A, Gregg D, Xie D, Saddler J.(2005) Enzymatic hydrolysis of steam-exploded and ethanol organosolv-pretreated Douglas-Fir by novel and commercial fungal cellulases. Appl Biochem Biotechnol.121-124: 219-30).

Cellobiohydrolase sequences of the glucohydrolase class 7 (cel7) are known to the art from several fungal sources. The Talaromyces emersonii Cel7 cellobiohydrolase is known and expression was reported in Escherichia coli. Grassick *et al.* present a report on the purification and 3D structural determination of a native core CBH protein, and of the cloning and over-expression of the corresponding gene, from a thermophilic fungal source. CBH 16 was found to be extremely thermostable with a temperature optimum of 68 °C at pH 5.0 and a half-life (t½) of 68.0 min at 80 °C and pH 5.0. (Grassick A, Murray PG, Thompson R, Collins CM, Byrnes L, Birrane G, Higgins TM, Tuohy MG. Three-dimensional structure of a thermostable native cellobiohydrolase, CBH IB, and molecular characterization of the cel7 gene from the filamentous fungus, Talaromyces emersonii. Eur J Biochem. 2004 Nov;271 (22):4495-4506) and Saccharomyces cerevisiae (Voutilainen SP, Murray PG, Tuohy MG, Koivula A. Expression of Talaromyces emersonii cellobiohydrolase Cel7A in Saccharomyces cerevisiae and rational mutagenesis to improve its thermostability and activity. Protein Eng Des Sel. 2010 Feb;23(2):69-79), however the protein was either produced in inactive form or at rather low yields (less or equal to 5mg/l). Hypocrea jecorina cellobiohydrolase I can be produced from wild type or engineered strains of the genus Hypocrea or Trichoderma at high yields. Improved sequences of Hypocrea jecorina Cel7A are disclosed by US7459299B2, US7452707B2, WO2005/030926, WO01/04284A1 or US2009/0162916 A1.

Positions leading to improvements were deduced from alignments with sequences from reported thermostable enzymes, suggested from structural information and shuffling of identified positions followed by limited screenings. Screening of larger libraries in transformable organisms such as *Saccharomyces cerevisiae* was reported by application of very sensitive fluorescent substrates, which resemble native substrates in a very restricted way. (Percival Zhang YH, Himmel ME, Mielenz JR. Outlook for cellulase improvement: screening and selection strategies. Biotechnol Adv. 2006 Sep-Oct;24(5):452-81).

The production of cellobiohydrolases from other fungal systems such as *Thermoascus aurantiacus, Chrysosporium lucknowense* or Phanerochaete chrysosporium was reported. Expression of Cel7 cellobiohydrolase from yeasts was reported, but enzymatic yields or enzyme properties remain unsatisfactory. (Penttilä ME, André L, Lehtovaara P, Bailey M, Teeri TT, Knowles JK. Efficient secretion of two fungal cellobiohydrolases by Saccharomyces cerevisiae. Gene. 1988;63(1):103-12).

WO03/000941 discloses a number of CBHs and their corresponding gene sequences. Physiological properties and applications however were not disclosed. The fusion of cellulose binding domains to catalytic subunits of cellobiohydrolases is reported to improve the hydrolytic properties of proteins without a native domain.

US 2009042266 (A1**)** discloses fusions of Thermoascus aurantiacus Cel7A with cellulose binding domains from cellobiohydrolase I from *Chaetomium thermophilum* and *Hypocrea jecorina*.

US5686593 reports the fusion of specially designed linker regions and binding domains to cellobiohydrolases.

**Hong et al. (2003)** describe the production of *Thermoascus aurantiacus* CBHI in yeast and its characterization. (Hong J, Tamaki H, Yamamoto K, Kumagai H Cloning of a gene encoding thermostable cellobiohydrolase from Thermoascus aurantiacus and its expression in yeast. Appl Microbiol Biotechnol. 2003 Nov;63(1):42-50).

**Tuohy et al. (2002)** report the expression and characterization of *Talaromyces emersonii* CBH. (Tuohy MG, Walsh DJ, Murray PG, Claeyssens M, Cuffe MM, Savage AV, Coughlan MP.:Kinetic parameters and mode of action of the cellobiohydrolases produced by Talaromyces emersonii. Biochim Biophys Acta. 2002 Apr 29;1596(2):366-80).

**Nevoigt et al. (2008)** reports on the expression of cellulolytic enzymes in yeasts. (Nevoigt E. Progress in metabolic engineering of Saccharomyces cerevisiae. Microbiol Mol Biol Rev. 2008 Sep;72(3):379-412).

**Fujita et al. (2004)** reports on a *Saccharomyces cervisiae* strain expressing a combination of an endoglucanase, a beta glucosidase and a CBHII displayed on the cell surface. Cellobiohydrolase I (Cel7) was not used in this setup. (Fujita Y, Ito J, Ueda M, Fukuda H, Kondo A. Synergistic saccharification, and direct fermentation to ethanol, of amorphous cellulose by use of an engineered yeast strain codisplaying three types of cellulolytic enzyme. Appl Environ Microbiol. 2004 Feb;70(2):1207-12).

**Boer H et al. (2000)** describes the expression of GH7 classified enzymes in different yeast hosts but expressed protein levels were low. (Boer H, Teeri TT, Koivula A. Characterization of Trichoderma reesei cellobiohydrolase Cel7A secreted from Pichia pastoris using two different promoters. Biotechnol Bioeng. 2000 Sep 5;69(5):486-94).

**Godbole et al (1999)** and **Hong et al ( 2003)** found that proteins of this enzyme class expressed fom yeast were often misfolded, hyperglycosylated and hydrolytic capabilities decreased compared to the protein expressed from the homologous host. (Godbole S, Decker SR, Nieves RA, Adney WS, Vinzant TB, Baker JO, Thomas SR, Himmel ME. Cloning and expression of Trichoderma reesei cellobiohydrolase I in Pichia pastoris. Biotechnol Prog. 1999 Sep-Oct;15(5):828-33).

**Kanokratana et al (2008)**, **Li et** al **(2009)** as well as CN01757710 describe the efficient expression of Cel7 CBH I enzymes, however these proteins are lacking celllulose binding domains required for efficient substrate processing. (Kanokratana P, Chantasingh D, Champreda V, Tanapongpipat S, Pootanakit K, Eurwilaichitr L Identification and expression of cellobiohydrolase (CBHI) gene from an endophytic fungus, Fusicoccum sp. (BCC4124) in Pichia pastoris. LProtein Expr Purif. 2008 Mar;58(1):148-53. Epub 2007 Sep 19; Li YL, Li H, Li AN, Li DC. Cloning of a gene encoding thermostable cellobiohydrolase from the thermophilic fungus Chaetomium thermophilum and its expression in Pichia pastoris. J Appl Microbiol. 2009 Jun;106(6):1867-75).

**Voutilainen (2008)** and **Viikari (2007)** disclose Cel7 enzymes comprising thermostable cellobiohydrolases, however with only low to moderate expression levels from *Trichoderma reesei,* (Voutilainen SP, Puranen T, Siika-Aho M, Lappalainen A, Alapuranen M, Kallio J, Hooman S, Viikari L, Vehmaanperä J, Koivula A. Cloning, expression, and characterization of novel thermostable family 7 cellobiohydrolases. Biotechnol Bioeng. 2008 Oct 15;101(3):515-28. PubMed PMID: 18512263; Viikari L, Alapuranen M, Puranen T, Vehmaanperä J, Siika-Aho M. Thermostable enzymes in lignocellulose hydrolysis. Adv Biochem Eng Biotechnol. 2007;108:121-45).

**Grassick et al. (2004)** disclose unfolded expression of Cellobiohydrolase I from *Talaromyces emersonii* in *Escherichia coli* but not in yeast. (Grassick A, Murray PG, Thompson R, Collins CM, Byrnes L, Birrane G, Higgins TM, Tuohy MG. Three-dimensional structure of a thermostable native cellobiohydrolase, CBH IB, and molecular characterization of the cel7 gene from the filamentous fungus, Talaromyces emersonii. Eur J Biochem. 2004 Nov;271(22):4495-506).

WO 2009/138877 describes a method for heterologous expression of polypeptides encoded by wild-type and codon-optimized variants of cbhl and/or cbh2 from the fungal organisms *Talaromyces emersonii (T. emersonii), Humicola grisea (H. grisea), Thermoascus aurantiacus (T. aurantiacus),* and *Trichoderma reesei (T. reesei)* in host cells, such as the yeast *Saccharomyces cerevisiae*. The expression in such host cells of the corresponding genes, and variants and combinations thereof, were found to result in improved specific activity of the expressed cellobiohydrolases.

WO 2009/139839 describes a methods and composition for a large capacity alphavirus vector and particle. In some aspects methods for providing alphavirus particles comprising a modified capsid protein are described.

Therefore, there is a need for cellulase enzymes with improved characteristics for the use in technical processes for cellulose hydrolysis. In particular there is a need for CBH enzymes with higher catalytic activity and/or higher stability under process conditions. Moreover there is a need for CBH enzymes with higher productivity in fungal and/or yeast expression and secretion systems.

### Summary of the invention

The present invention provides a polypeptide having cellobiohydrolase activity. This polypeptide comprises an amino acid sequence having at least 85 % sequence identity to SEQ ID NO: 2. wherein the amino acid residue at position Q1 of SEQ ID NO : 2 is modified by substitution or deletion.

Furthermore, the present invention discloses a nucleic acid encoding the polypeptide of the present invention, preferably having at least 95 % identity to SEQ ID NO: 1, a vector comprising this nucleic acid and a host transformed with said vector.

The present application further discloses a method of producing a cellobiohydrolase protein encoded by a vector of the present invention, a method for identifying polypeptides having cellobiohydrolase activity, and a method of preparing such polypeptides having cellobiohydrolase activity.

The present invention also provides a polypeptide having cellobiohydrolase activity, wherein the polypeptide comprises an amino acid sequence having at least 85 % sequence identity to SEQ ID NO: 2, wherein the amino acid residue at position Q1 of SEQ ID NO : 2 is modified by substitution or deletion, wherein one or more of the following amino acid residues of the sequence defined by SEQ ID NO: 2 are modified by substitution or deletion: G4, A6, T15, Q28, W40, D64, E65, A72, S86, K92, V130, V152, Y155, K159, D181, E183, N194, D202, P224, T243, Y244, I277, K304, N310, S311, N318, D320, T335, T344, D346, Q349, A358, Y374, A375, T392, T393, D410, Y422, P442, N445, R446, T456, S460, P462, G463, H468 and/or V482 of amino acids 1 to 500 of SEQ ID NO: 2.

Moreover, the present application discloses a polypeptide having cellobiohydrolase activity, which is obtainable by the method of preparing a polypeptide having cellobiohydrolase activity according to the present application, and a polypeptide having cellobiohydrolase activity, wherein the polypeptide comprises an amino acid sequence having at least 80 % sequence identity to SEQ ID NO: 5, wherein one or more of the following amino acid residues of the sequence defined by SEQ ID NO: 5 are modified by substitution or deletion: Q1, G4, A6, T15, Q28, W40, D64, E65, A72, S86, K92, V130, V152, Y155, K159, D181, E183, N194, D202, P224, T243, Y244, I277, K304, N310, S311, N318, D320, T335, T344, D346, Q349, A358, Y374, A375, T392, T393, D410 and/or Y422 of amino acids 1 to 440 of SEQ ID NO: 5.

The present application furthermore discloses a polypeptide having cellobiohydrolase activity comprising an amino acid sequence having at least 85 % sequence identity to SEQ ID NO: 12 wherein one or more of the following amino acid residues of the sequence defined by SEQ ID NO: 12 are modified by substitution or deletion: Q1, T15, Q28, W40, C72, V133, V155, Y158, T162, Y247, N307, G308, E317, S341, D345, Y370, T389, Q406, N441, R442, T452, S456, P458, G459, H464 and/or V478.

The present invention furthermore discloses a composition comprising a polypeptide of the present invention and one or more endoglucanases and/or one or more beta-glucosidases and/or one or more further cellobiohydrolases and/or one or more xylanases.

The present invention further provides the use of a polypeptide or the composition of the present invention for the enzymatic degradation of lignocellulosic biomass, and/or for textiles processing and/or as ingredient in detergents and/or as ingredient in food or feed compositions.

### Brief description of the figures

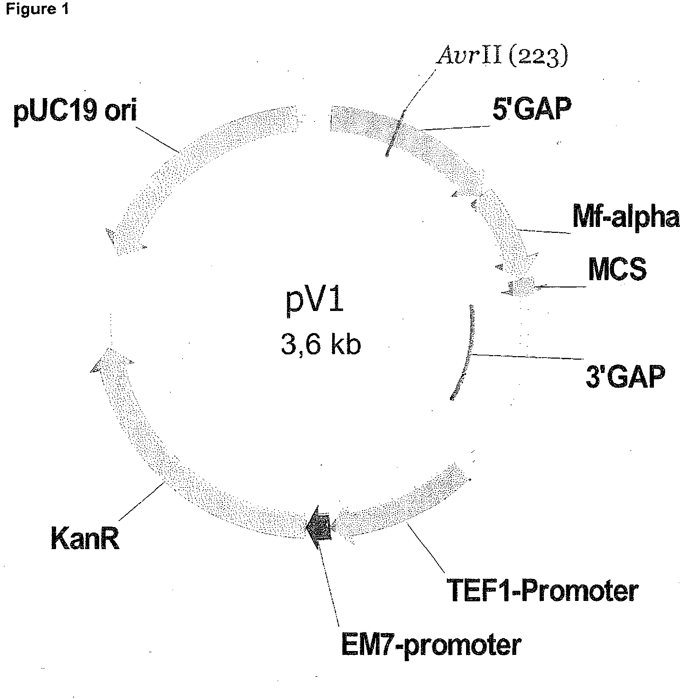

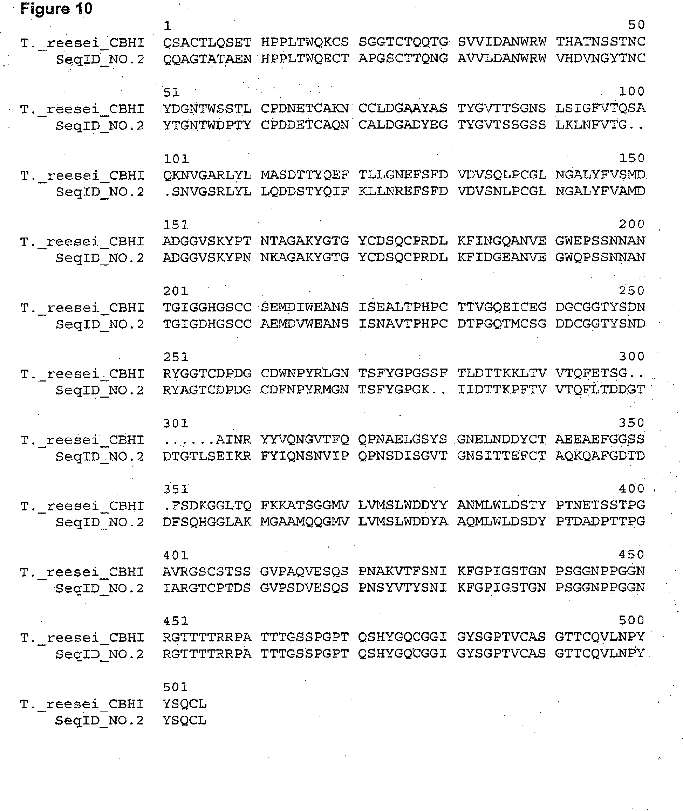

Figure 1: Restriction Maps of pV1 for constitutive expression of Proteins in *Pichia pastoris:* pUC19 - ori: Origin of replication in E. coli; KanR: Kanamycine/G418 Resistance with TEF1 and EMZ Promoter sequences for selection in *Pichia pastoris* and *E*. *coli*, respectively; 5'GAP: glyceraldehyde-3-phosphate dehydrogenase Promoter region; 3'-GAP: terminator region; SP MFalpha: *Saccharomyces cerevisiae* mating factor alpha signal sequence; MCS: multiple cloning site.

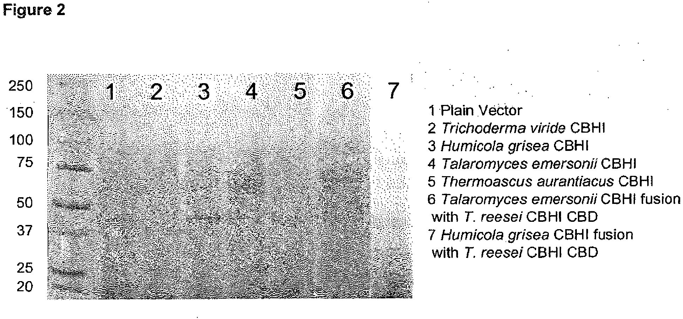

Figure 2: Commassie stained SDS-PAGE of 10-fold concentrated supernatants of shake-flask cultures of *Pichia pastoris* CBS 7435 containing expression plasmids with coding sequences for the mature CBHI proteins of *Trichoderma viride* (CBH-f; lane 2), *Humicola grisea* (CBH-d; lane 3), *Talaromyces emersonii* (CBH-b; lane 4), *Thermoascus aurantiacus* (CBH-e; lane 5), as well as the *Talaromyces emersonii* CBHI-CBD fusion (CBH-a; lane 6) and the *Humicola grisea*-CBD fusion (CBH-g; lane 7) in N-terminal fusion to the signal peptide of the *Saccharomyces cerevisiae* mating factor alpha under control of the *Pichia pastoris* glyceraldehyde-3-phosphate dehydrogenase promoter.

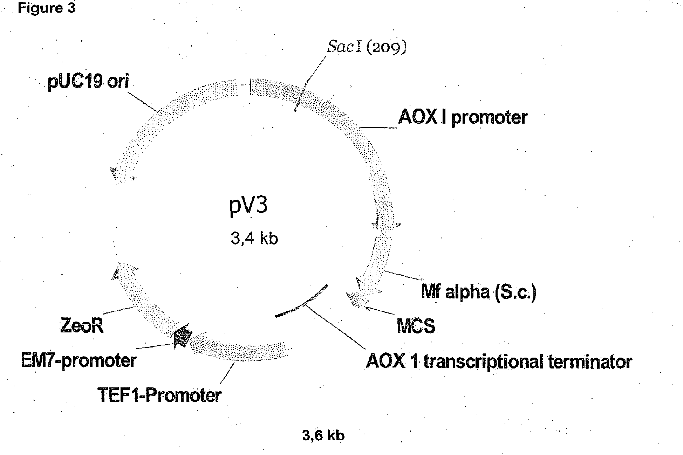

Figure 3: Map of the pV3 expression plasmid for protein expression in *Pichia pastoris.* Replicons: pUC19 - ori: Origin of replication in E. coli; ZeoR: Zeocine resistance gene with TEF1 and EM7 promoter sequences for expression in *Pichia pastoris* and *E. coli,* respectively; AOX I promoter: Promoter region of the Pichia pastoris alcohol oxidase I gene; AOX 1 transcriptional terminator: terminator region; SP MFalpha: *Saccharomyces cerevisiae* mating factor alpha signal sequence; MCS: multiple cloning site.

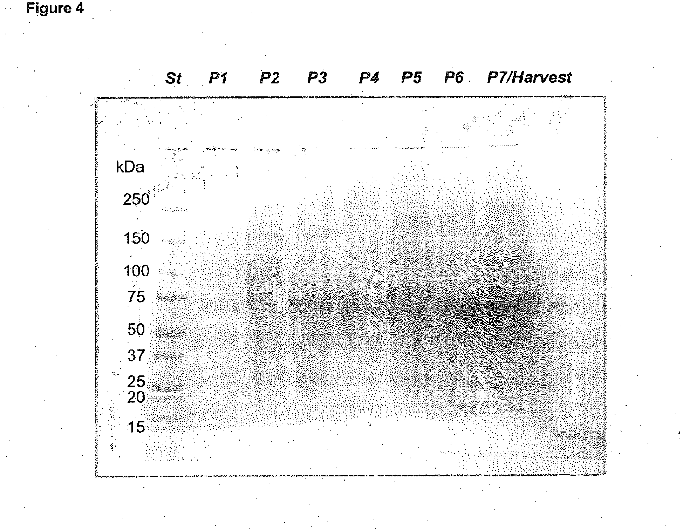

Figure 4: SDS-PAGE analysis of culture supernatant samples taken from the fermentation of a *Pichia pastoris* strain with a genomic integration of an AOXI-expression cassette, expressing the *Talaromyces emersonii* CBHI / *Trichoderma reesei* -CBD fusion peptide (CBH-a) in a 71 bioreactor during methanol induction. Samples P1 - P7 are taken at the beginning of the methanol induction and after 20, 45, 119.5, 142.5, 145.5 and 167 hours, respectively.

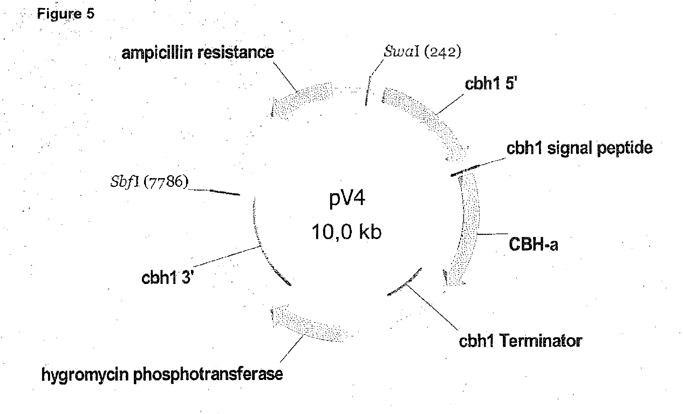

Figure 5: Map of pV4 expression plasmid for the expression of the *Talaromyces emersonii* CBHI / *Trichoderma reesei*-CBD fusion peptide (CBH-ah) in *Trichoderma reesei.* Replicon: pUC19 for replication in *E. coli.* cbh1 5': 5' promoter region of the *Trichoderma* CBHI gene; cbh1 signal peptide: Coding sequence for the *Trichoderma reesei* CBHI leader peptide; CBH-a: *Talaromyces emersonii* CBHI / *Trichoderma reesei* -CBD fusion peptide: coding region for SEQ ID NO: 18; cbh1 Terminator: 3' termination region of the *Trichoderma reesei* CBHI locus; hygromycine resistance: coding region of the hygromycine phosphotransferase under control of a *Trichoderma reesei* phosphoglycerate kinase promoter; cbh1 3': homology sequence to the termination region of the *Trichoderma reesei* CBHI locus for double crossover events.

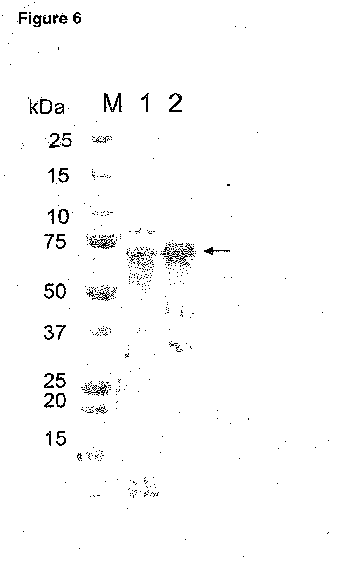

Figure 6: SDS-Page of *Trichoderma reesei* culture supernatants. Lane 1 shows the expression pattern of a replacement strain carrying a *Talaromyces emersonii* CBHI / *Trichoderma reesei* -CBD fusion (CBH-ah) inplace of the *native* CBHI gene. In comparison lane 2 shows the pattern for the unmodified strain under same conditions. M: molecular size marker.

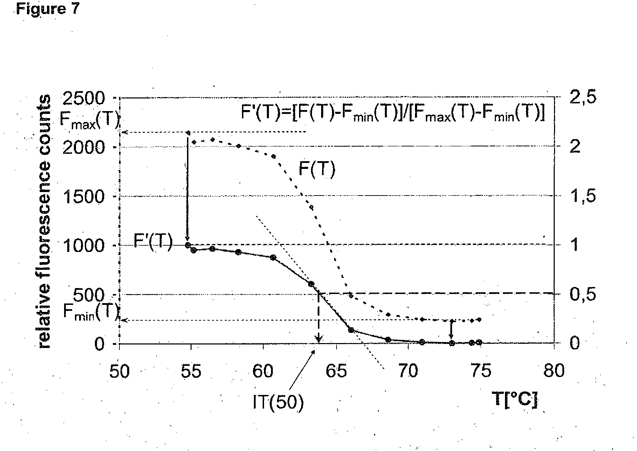

Figure 7: Determination of IT50 values from Substrate Conversion Capacity vs. temperature graphs after normalization. For the normalization step the maximum and the minimum fluorescence values for the selected temperature are correlated to 1 or 0, respectively. Linear interpolation to F'(T)=0.5 between the nearest two temperature points with normalized values next to 0.5 results in the defined IT50 temperature.

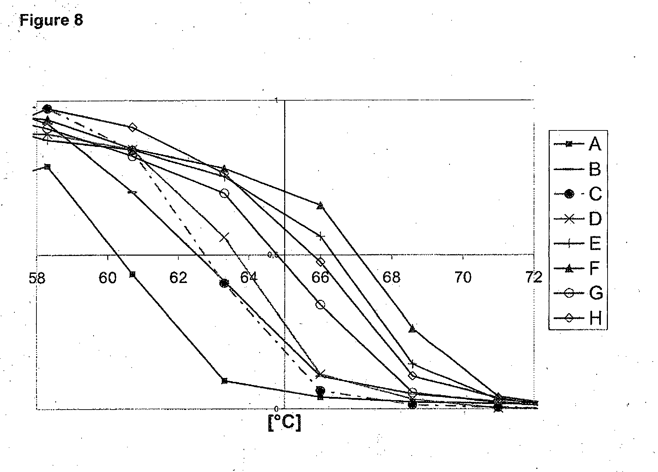

Figure 8: Normalized Conversion Capacity vs. temperature graphs of "wt" *Talaromyces emersonii* CBHI / *Trichoderma reesei* -CBD fusions (CBH-ah: SEQ ID NO: 18 = SEQ ID NO: 2 + 6x His-Tag) and mutants based on 4-Methylumbelliferyl -β-D-lactoside hydrolysis results evaluated at various temperatures. The fluorescence values were normalized according to figure 8 over the temperature range from 55°C to 75°C.
   A...wt;
   B...G4C,A72C;
   C...G4C,A72C,Q349K;
   D...G4C,A72C,D181N,Q349K;
   E...Q1L,G4C,A72C,D181N,E183K,Q349R;
   F...QL,G4C,A72C,S86T,D181N,E183K,D320V,Q349R;
   G...G4C, A72C,E183K,D202Y,N310D,Q349R;
   H ... Q1L,G4C,A72C, A145T,H203R,Q349K,T403K;

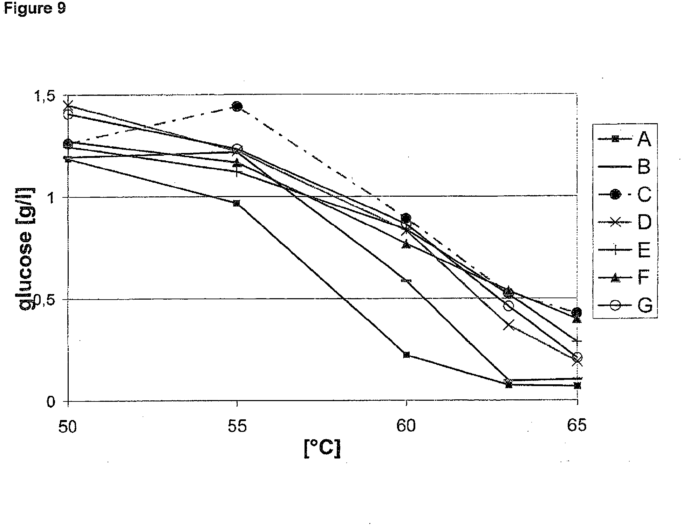

Figure 9: Glucose yields of hydrolysis of pretreated straw with wt and mutated *Talaromyces emersonii* CBHI / *Trichoderma reesei* -CBD (CBH-ah) fusion protein after hydrolysis for 48 hours in the presence of a β-glycosidase. The variants are characterized by the following mutations with respect to SEQ ID NO: 18 and were expressed from *Pichia pastoris* in shake flask cultures and isolated from the supernatant by affinity chomatography using Ni-NTA.
   A: wt
   B: G4C,A72C
   C: G4C,A72C,Q349K
   D: G4C,A72C, D181N,Q349K
   E: Q1L,G4C,A72C,D181N,E183K,Q349R
   F: Q1L,G4C,A72C,S86T,D181N,E183K,D320V,Q349R
   G: G4C, A72C, E183K,D202Y,N310D,Q349R
Figure 10: Alignment of SEQ ID NO: 2 with the *Trichoderma reesei* CBHI. The alignment matrix blosum62mt2 with gap opening penalty of 10 and gap extension penalty of 0.1 was used to create the alignment.

### Detailed description of the invention

The present invention discloses a polypeptide having cellobiohydrolase activity, which comprises an amino acid sequence with at least 85 % sequence identity to SEQ ID NO: 2 wherein the amino acid residue at position Q1 of SEQ ID NO : 2 is modified by substitution or deletion. "Cellobiohydrolase" or "CBH" refers to enzymes that cleave cellulose from the end of the glucose chain and produce cellobiose as the main product. Alternative names are 1,4-beta-D-glucan cellobiohydrolases or cellulose 1,4-beta-cellobiosidases. CBHs hydrolyze the 1,4-beta-D-glucosidic linkages from the reducing or non-reducing ends of a polymer containing said linkages. "Cellobiohydrolase I" or "CBH I" act from the reducing end of the cellulose fiber. "Cellobiohydrolase II" or "CBH II" act from the non-reducing end of the cellulose fiber. Cellobiohydrolases typically have a structure consisting of a catalytic domain and one or more "cellulose-binding domains" or "CBD". Such domains can be located either at the N- or C-terminus of the catalytic domain. CBDs have carbohydrate-binding activity and they mediate the binding of the cellulase to crystalline cellulose and presence or absence of binding domains are known to have a major impact on the processivity of an enzyme especially on polymeric substrates.

The parental sequence is given in SEQ ID NO: 2. The sequence derives from the C-terminal fusion of the linker domain and cellulose binding domain of *Trichoderma reesei* CBHI (SEQ ID NO: 4) to the catalytic domain of *Talaromyces* emersonii CBHI (SEQ ID NO: 5).

In a preferred aspect, the invention discloses protein variants that show a high activity at high temperature over an extended period of time. Preferably, the polypeptide of the present invention maintains 50 % of its maximum substrate conversion capacity when the conversion is done for 60 minutes at a temperature of 60 °C or higher. The respective temperature is also referred to as the IT50 value. In other words, the IT50 value is preferably 60 °C or higher. "Substrate Conversion Capacity" of an enzyme is herein defined as the degree of substrate conversion catalyzed by an amount of enzyme within a certain time period under defined conditions (Substrate concentration, pH value and buffer concentration, temperature), as can be determined by end-point assaying of the enzymatic reaction under said conditions. "Maximum Substrate Conversion Capacity" of an enzyme is herein defined as the maximum in Substrate Conversion Capacity found for the enzyme within a number of measurements performed as described before, where only one parameter, e.g. the temperature, was varied within a defined range. According to the present invention, the assay described in Example 8 is used to determine these parameters.

Furthermore, the disclosed polypeptides have preferably an IT50 value in the range of 62 to 70 °C, more preferably 65 to 70 °C.

The polypeptide of the present invention preferably comprises an amino acid sequence having at least 90 %, preferably at least 95 %, more preferably at least 99 % sequence identity to SEQ ID NO: 2, wherein the amino acid residue at position Q1 of SEQ ID NO : 2 is modified by substitution or deletion. Furthermore, it is particularly preferred that the amino acid sequence of the polypeptide has the sequence as defined by SEQ ID NO: 2, wherein the amino acid residue at position Q1 of SEQ ID NO : 2 is modified by substitution or deletion, or a sequence as defined by SEQ ID NO: 2 wherein the amino acid residue at position Q1 of SEQ ID NO : 2 is modified by substitution or deletion wherein 1 to 75, more preferably 1 to 35 amino acid residues are substituted, deleted, or inserted.

Particularly preferred are variants of the protein of SEQ ID NO: 2, wherein the amino acid residue at position Q1 of SEQ ID NO : 2 is modified by substitution or deletion. "Protein variants" are polypeptides whose amino acid sequence differs in one or more positions from this parental protein, whereby differences might be replacements of one amino acid by another, deletions of single or several amino acids, or insertion of additional amino acids or stretches of amino acids into the parental sequence. Per definition variants of the parental polypeptide shall be distinguished from other polypeptides by comparison of sequence identity (alignments) using the ClustalW Algorithm (Larkin M.A., Blackshields G., Brown N.P., Chenna R., McGettigan P.A., McWilliam H., Valentin F., Wallace I.M., Wilm A., Lopez R., Thompson J.D., Gibson T.J. and Higgins D.G. (2007) ClustalW and ClustalX version 2. Bioinformatics 2007 23(21): 2947-2948). Methods for the generation of such protein variants include random or site directed mutagenesis, site-saturation mutagenesis, PCR-based fragment assembly, DNA shuffling, homologous recombination in-vitro or in-vivo, and methods of gene-synthesis.

The nomenclature of amino acids, peptides, nucleotides and nucleic acids is done according to the suggestions of IUPAC. Generally amino acids are named within this document according to the one letter code.

Exchanges of single amino acids are described by naming the single letter code of the original amino acid followed by its position number and the single letter code of the replacing amino acid, i.e. the change of glutamine at position one to a leucine at this position is described as "Q1L". For deletions of single positions from the sequence the symbol of the replacing amino acid is substituted by the three letter abbreviation "del" thus the deletion of alanine at position 3 would be referred to as "A3del". Inserted additional amino acids receive the number of the preceding position extended by a small letter in alphabetical order relative to their distance to their point of insertion. Thus, the insertion of two tryptophanes after position 3 is referred to as "3aW, 3bW". Introduction of untranslated codons TAA, TGA and TAG into the nucleic acid sequence is indicated as "*" in the amino acid sequence, thus the introduction of a terminating codon at position 4 of the amino acid sequence is referred to as "G4*".

Multiple mutations are separated by a plus sign or a slash or a comma. For example, two mutations in positions 20 and 21 substituting alanine and glutamic acid for glycine and serine, respectively, are indicated as "A20G+E21S" or "A20G/E21S" "A20G,E21S".

When an amino acid residue at a given position is substituted with two or more alternative amino acid residues these residues are separated by a comma or a slash. For example, substitution of alanine at position 30 with either glycine or glutamic acid is indicated as "A20G,E" or "A20G/E", or "A20G, A20E".

When a position suitable for modification is identified herein without any specific modification being suggested, it is to be understood that any amino acid residue may be substituted for the amino acid residue present in the position. Thus, for instance, when a modification of an alanine in position 20 is mentioned but not specified, it is to be understood that the alanine may be deleted or substituted for any other amino acid residue (i.e. any one of R, N, D, C, Q, E, G, H, I, L, K, M, F, P, S, T, W, Y and V).

The terms "similar mutation" or "similar substitution" refer to an amino acid mutation that a person skilled in the art would consider similar to a first mutation. Similar in this context means an amino acid that has similar chemical characteristics. If, for example, a mutation at a specific position leads to a substitution of a non-aliphatic amino acid residue (e.g. Ser) with an aliphatic amino acid residue (e.g. Leu), then a substitution at the same position with a different aliphatic amino acid (e.g. Ile or Val) is referred to as a similar mutation. Further amino acid characteristics include size of the residue, hydrophobicity, polarity, charge, pK-value, and other amino acid characteristics known in the art. Accordingly, a similar mutation may include substitution such as basic for basic, acidic for acidic, polar for polar etc. The sets of amino acids thus derived are likely to be conserved for structural reasons. These sets can be described in the form of a Venn diagram (Livingstone CD, and Barton GJ. (1993) "Protein sequence alignments: a strategy for the hierarchical analysis of residue conservation" Comput.Appl Biosci. 9: 745-756; Taylor W. R. (1986) "The classification of amino acid conservation" J.Theor.Biol. 119; 205-218). Similar substitutions may be made, for example, according to the following grouping of amino acids: Hydrophobic: F W Y H K M I L V A G; Aromatic: F W Y H; Aliphatic: I L V; Polar: W Y H K R E D C S T N; Charged H K R E D; Positively charged: H K R; Negatively charged: E D.

As convention for numbering of amino acids and designation of protein variants for the description of protein variants the first glutamine (Q) of the amino acid sequence QQAGTA within the parental protein sequence given in SEQ ID NO: 2 is referred to as position number 1 or Q1 or glutamine 1. The numbering of all amino acids will be according to their position in the parental sequence given in SEQ ID NO: 2 relative to this position number 1.

The present invention furthermore discloses variants of the polypeptides of the present invention with changes of their sequence at one or more of the positions : G4, A6, T15, Q28, W40, D64, E65, A72, S86, K92, V130, V152, Y155, K159, D181, E183, N194, D202, P224, T243, Y244, I277, K304, N310, S311, N318, D320, T335, T344, D346, Q349, A358, Y374, A375, T392, T393, D410, Y422, P442, N445, R446, T456, S460, P462, G463, H468 and/or V482 of amino acids 1 to 500 of SEQ ID NO: 2, wherein the amino acid residue at position Q1 of SEQ ID NO : 2 is modified by substitution or deletion.

In a preferred embodiment, the variant of the polypeptides of the present invention comprises one or more specific changes of their sequence at the following positions (preferred exchange) or a similar mutation.

| Position | Preferred Exchange | Similar mutation |
|---|---|---|
| Q1 | L | I, L, V, A, G, |
| G4 | C | C, D, E, R, H, K |
| A6 | G, V | I, L, V, G |
| T15S | S | Q, N |
| Q28 | R | H, K, R |
| W40 | R | H, K, R |
| D64 | N | Q, N, S, T, E |
| E65 | K, V | H, K, R |
| A72 | C, V | M, I, L, V, G, C |
| S86 | T | H, K, R, E, D, C, T, N |
| K92 | R | H, R |
| V130 | I | F, W, Y, H, K, M, I, L, A, G |
| V152 | A, E | D, E, A, I, L, G, P |
| Y155 | C | C, D, E, R, H |
| K159 | E | D, E |
| D181 | N | Y, H, K, R, C, S, T, N |
| E183 | V, K | I, L, V, A, G, R, K, H |
| N194 | C, R, Y, D, K, I, L, G, Q, S, V | F, W, C, R, Y, D, K, I, L, G, Q, S, V, H |
| D202 | Y, N, G | A, R, N, C, Q, G, H, I, L, K, M, F, P, S, T, W, Y, V |
| P224 | L | I, L, V, A, G |
| T243 | I, C, R, Y, A, F, Q, P,D, V, W, L, M | H, K, I, C, R, Y, A, F, Q, P, D, V, W, L, M |
| Y244 | F, H | F, W, H, |
| I277 | V | G, A |
| K304 | R | H, R |
| N310 | D | C, D, E, R, H |
| S311 | G, N | I, L, V, A, G, Q, T, D, |
| N318 | Y | F, W, Y, H |
| D320 | V, E, N | W, Y, H, K, R, E, C, S, T, N |
| T335 | I | F, W, Y, H, K, M, I, L, V, A, G |
| T344 | M | F, W, Y, H, K, M, I, L, V, A, G |
| D346 | G, A, E | I, L, V, A, G |
| Q349 | R, K | H, K, R |
| A358 | E | F, W, Y, H, K, M, I, L, V, G |
| Y374 | C, P, R, H, S, A | C, P, R, H, S, A, K, E, D, C, F |
| A375 | C, D, N, Y, R, G, L, V, E, G, T, M | H, K, C, D, N, Y, R, Q, L, V, E, G, T, M |
| T392 | C, D, K | H, K, R, E, D, C |
| T393 | A | I, L, V, A, G, |
| D410 | G | F, W, Y, H, K, M, I, L, V, A, G |
| Y422 | F | H, F, W |
| P442 | S, del | S, del, T |
| N445 | D | D, E, Q |
| R446 | S, G | A, S, G, V, I, L, C, P |
| T456 | A | S, A, G, I, L, V, C, P |
| S460 | L, P | G, A, I, L, P, V |
| P462 | L, del | G, A, I, L, V, del |
| G463 | D | D, E |
| H468 | L, Q, R | K, R, E, D, C, S, T, N |
| V482 | A, I | G, L, A, I |

Even more preferably, the variant of the polypeptides of the present invention comprises an amino acid sequence selected from the sequences with mutations with respect to SEQ ID NO: 2, wherein the amino acid residue at position Q1 of SEQ ID NO : 2 is modified by substitution or deletion, optionally fused with a C-terminal 6x-His Tag, listed in the following Table.

| | Mutations with respect to SEQ ID NO: 2 |
|---|---|
| 75 | Q1L,G4C,A72C,Q349K |
| 88 | Q1L,G4C,A72C,S86T,Q349R |
| 89 | Q1L,G4C,A72C,D181N,Q349R |
| 90 | Q1L,G4C,A72C,E183K,Q349R |
| 91 | Q1L,G4C,A72C,D181N,E183K,Q349R |
| 92 | Q1L,G4C,A72C,D320V,Q349R |
| 93 | Q1L,G4C,A72C,S86T,D181N,E183K,D320V,Q349R |
| 98 | Q1L,G4C,A72C,Q349R |
| 148 | Q1L,G4C,A72C,Q349K,T392M |
| 153 | Q1L,G4C,A68T,A72C,Q349K,G439D,R453S |
| 154 | Q1L,G4C,A72C,D202N,Q349K |
| 155 | Q1L,G4C,A68T,A72C,Q349K |
| 156 | Q1L,G4C,A72C,K154R,Q349K,T3931 |
| 157 | Q1L,G4C,A72C,S193P,Q349K,V482I |
| 158 | Q1L,G4C,A72C,H203R,Q349K,P442S |
| 159 | Q1L,G4C,A72C,Q349K,H468R |
| 160 | Q1L,G4C,A72C,D202N,Q349K,G486D |
| 161 | Q1L,G4C,E65K,A72C,Q349K |
| 162 | Q1L,G4C,A72C,Q349K,Y422F |
| 163 | Q1L,G4C,Q28R,A72C,Q349K,H468L |
| 164 | Q1L,G4C,A72C,D181N,D247N,Q349K |
| 165 | Q1L,G4C,A72C,D181N,Q349K,T451S |
| 166 | Q1L,G4C,Q28R,A72C,Q349K |
| 167 | Q1L,G4C,A72C,A145T,H203R,Q349K,T403K |
| 168 | Q1L,G4C,A72C,I200F,Q349K,L500I |
| 169 | Q1L,G4G,D64N,A72C,Q349K |
| 170 | Q1L,G4C,A72C,V152A,Q349K |
| 171 | Q1L,G4C,T15S,A72C,Y244F,Q349K |
| 172 | Q1L,G4C,A6V,A72C,Q349K |
| 173 | Q1L,G4C,A72C,S311N,Q349K,G463D |
| 174 | Q1L,G4C,A72C,Y155C,0349K |
| 175 | Q1L,G4C,A72C,S311N,Q349K |
| 176 | Q1L,G4C,A72C,D346V,Q349K |
| 177 | Q1L,G4C,A72C,Q349K,T392K |
| 178 | Q1L,G4C,A72C,S311G,Q349K |
| 179 | Q1L,G4C,A72C,S311G,Q349K,H468R |
| | |

In a further aspect, the present invention discloses a nucleic acid encoding the polypeptide of the present invention. The nucleic acid is a polynucleotide sequence (DNA or RNA) which is, when set under control of an appropriate promoter and transferred into a suitable biological host or chemical environment, processed to the encoded polypeptide, whereby the process also includes all post-translational and post-transcriptional steps necessary. The coding sequence can be easily adapted by variation of degenerated base-triplets, alteration of signal sequences, or by introduction of introns, without affecting the molecular properties of the encoded protein. The nucleic acid of the present invention has preferably at least 95 %, more preferably at least 97 %, and most preferably 100% identity to SEQ ID NO: 1. The present invention also provides a vector comprising this nucleic acid and a host transformed with said vector.

The present application also discloses methods for the production of polypeptides of the present invention and variants thereof in various host cells, including yeast and fungal hosts. It also discloses the use of the resulting strains for the improvement of protein properties by variation of the sequence. Furthermore, the present application discloses methods for the application of such polypeptides in the hydrolysis of cellulose.

A further aspect of the application disdoses vectors and methods for the production of protein variants of SEQ ID NO: 2, wherein the amino acid residue at position Q1 of SEQ ID NO : 2 is modified by substitution or deletion, expressing them in yeast and testing their activity on cellulosic material by measuring the released mono- and/or oligomeric sugar molecules.

The present application further discloses a method of producing a cellobiohydrolase protein, comprising the steps:
a. obtaining a host cell, which has been transformed with a vector comprising the nucleic acid of the present invention;
b. cultivation of the host cell under conditions under which the cellobiohydrolase protein is expressed; and
c. recovery of the cellobiohydrolase protein.

In a preferred embodiment, the host cell is derived from the group consisting of Saccharomyces, Schizosaccharomyces, Kluyveromyces, Pichia, Hansenula, Aspergillus, Trichoderma, Penicillium, Candida and Yarrowina. The host cell is preferably capable of producing ethanol, wherein most preferred yeasts include *Saccharomyces cerevisiae, Pichia stipitis, Pachysolen tannophilus,* or a methylotrophic yeast, preferably derived from the group of host cells comprising *Pichia methanolica, Pichia pastoris, Pichia angusta, Hansenula polymorpha.*

It has surprisingly been found that the polypeptide according to the present invention and variants thereof can be expressed from yeast at high levels. "Yeast" shall herein refer to all lower eukaryotic organisms showing a unicellular vegetative state in their life cycle. This especially includes organisms of the class *Saccharomycetes,* in particular of the genus *Saccharomyces, Pachysolen, Pichia, Candida, Yarrowina, Debaromyces, Klyveromyces, Zygosaccharomyces.*

Thus, one aspect of the application discloses the expression of the claimed polypeptide and variants thereof in yeast. The efficient expression of this fusion protein (SEQ ID NO: 2) and derivative protein variants of SEQ ID NO: 2 from yeast can be achieved by insertion of the nucleic acid molecule of SEQ ID NO: 1 starting from nucleotide position 1 into an expression vector under control of at least one appropriate promoter sequence and fusion of the nucleotide molecule to an appropriate signal peptide, for example to the signal peptide of the mating factor alpha of *Saccharomyces cerevisiae.*

In a preferred embodiment, the polypeptide of the present invention and variants thereof are expressed and secreted at a level of more than 100 mg/l, more preferably of more than 200 mg/l, particularly preferably of more than 500 mg/l, or most preferably of more than 1 g/I into the supernatant after introduction of a nucleic acid encoding a polypeptide having an amino acid sequence with at least 85% sequence identity to the SEQ ID NO: 2 into a yeast. To determine the level of expression in yeast, the cultivation and isolation of the supernatant can be carried out as described in Example 3.

A further aspect of the application discloses methods for the production of a polypeptide according to the present invention in a filamentous fungus, preferably in a fungus of the genus Aspergillus or Trichoderma, more preferably in a fungus of the genus *Trichoderma,* most preferably in *Trichoderma reesei. "Filamentous fungi"* or *"fungi"* shall herein refer to all lower eukaryotic organisms showing hyphal growth during at least one state in their life cycle. This especially includes organisms of the *phylum Ascomycota* and *Basidiomycota,* in particular of the genus *Trichoderma, Talaromyces, Aspergillus, Penicillium, Chrysosporium, Phanerochaete, Thermoascus, Agaricus, Pleutrus, Irpex.* The polypeptide is expressed by fusion of the coding region of a compatible signal sequence to the nucleic acid molecule starting with nucleotide position 52 of SEQ ID NO: 3, as it was done in SEQ ID NO: 3 with the signal sequence of the *Trichoderma reesei* CBHI, and the positioning of the fusion peptide under control of a sufficiently strong promoter followed by transfer of the genetic construct to the host cell. Examples for such promoters and signal sequences as well as techniques for an efficient transfer have been described in the art.

In a further aspect the present application further discloses a method for identifying polypeptides having cellobiohydrolase activity, comprising the steps of:
a. Generating a library of mutant genes encoding mutant proteins by mutagenesis of a nucleic acid according to claim 9 or a nucleic acid having the sequence defined by SEQ ID NO: 6 (encoding SEQ ID NO: 5), preferably having the sequence defined by SEQ ID NO: 1;
b. Inserting each mutant gene into an expression vector;
c. Transforming yeast cells with each expression vector to provide a library of yeast transformants;
d. Cultivation of each yeast transformant under conditions under which the mutant protein is expressed and secreted;
e. Incubating the expressed mutant protein with a substrate;
f. Determining the catalytic activity of the mutant protein;
g. Selecting a mutant protein according to the determined catalytic activity.

Specifically, step d. may be performed by utilizing a well-plate format. This format preferably allows the high-throughput performance of the method for identifying polypeptides having cellobiohydrolase activity.

Preferably, the steps e. to g. of the method for identifying polypeptides having cellobiohydrolase activity are performed as follows:
e. Incubating the expressed mutant protein with cellulosic material;
f. Determining the amount of released sugar;
g. Selecting a mutant protein according to the amount of released sugar.

In another embodiment, the method for identifying polypeptides having cellobiohydrolase activity comprises the additional steps of:
h. Sequencing the selected mutant gene or protein;
i. Identifying the amino acid modification(s) by comparing the sequence of the selected mutant protein with the amino acid sequence of SEQ ID NO: 2.

The present application further discloses a method of preparing a polypeptide having cellobiohydrolase activity, comprising the steps:
a. Providing a polypeptide having cellobiohydrolase activity comprising an amino sequence having at least 70 % sequence identity to the catalytic domain of SEQ ID NO: 2 (SEQ ID NO: 5);
b. Identifying the amino acids of this polypeptide which correspond to the amino acids which are modified with respect to the amino acid sequence of SEQ ID NO: 2, as identified in step i. of the method for identifying polypeptides having cellobiohydrolase activity; and
c. Preparing a mutant polypeptide of the polypeptide provided in step a. by carrying out the amino acid modification(s) identified in step b. through site-directed mutagenesis.

Preferably, the polypeptide provided in step a. of the method of preparing a polypeptide having cellobiohydrolase activity is a wild type cellobiohydrolase derived from *Trichoderma reesei.*

The present application further discloses polypeptides having cellobiohydrolase activity, which are obtainable by the method of preparing a polypeptide having cellobiohydrolase activity according to the present application.

Furthermore, the present invention provides a composition comprising a polypeptide and/or variants thereof of the present invention and one or more cellulases, e.g. one or more endoglucanases and/or one or more beta-glucosidases and/or one or more further cellobiohydrolases and/or one or more xylanases. "Cellulases" or "Cellulolytic enzymes" are defined as enzymes capable of hydrolysing cellulosic substrates or derivatives or mixed feedstocks comprising cellulosic polymers. Such enzymes are referred to as having "cellulolytic activity", thus being able to hydrolyze cellulose molecules from such material into smaller oligo- or monosaccharides. Cellulolytic enzymes include cellulases and hemicellulases, in particular they include cellobiohydrolases (CBHs), endoglucanases (EGs) and beta-glucosidases (BGLs).

The present application further discloses a polypeptide having cellobiohydrolase activity, wherein the polypeptide comprises an amino acid sequence having at least 80 %, preferably at least 95%, more preferably at least 98%, even more preferably at least 99%, and most preferably 100% sequence identity to SEQ ID NO: 5, wherein one or more of the following amino acid residues of the sequence defined by SEQ ID NO: 5 are modified by substitution or deletion of: Q1, G4, A6, T15, Q28, W40, D64, E65, A72, S86, K92, V130, V152, Y155, K159, D181, E183, N194, D202, P224, T243, Y244, 1277, K304, N310, S311, N318, D320, T335, T344, D346, Q349, A358, Y374, A375, T392, T393, D410 and/or Y422 of amino acids 1 to 440 of SEQ ID NO: 5.

In a preferred embodiment, the polypeptide having cellobiohydrolase activity with an amino acid sequence having at least 80 % sequence identity to SEQ ID NO: 5 comprises one or more of the following modified amino acid residues of the sequence defined by SEQ ID NO: 5: Q1L, G4, A6G/V, T15S, Q28Q/R, W40R, D64N, E65K/V, A72V, S86T, K92K/R, V130I/V, V152A/E, Y155C, K159E, D181N, E183V/K, N194C/R/Y/D/K/I/UG/Q/S/V, D202Y/N/G, P224L, T243I/R/Y/A/F/Q/P/D/V/W/L/M, Y244F/H, I277V, K304R, N310D, S311G/N, N318Y, D320V/E/N, T335I, T344M, D346G/A/E/V, Q349R/K, A358E, Y374C/P/R/H/S/A, A375D/N/Y/R/Q/L/V/E/G/T/M, T392C/D/K, T393A, D410G, Y422F.

More preferably, the polypeptide having cellobiohydrolase activity comprises one or more modified amino acid residues of the sequence defined by SEQ ID NO: 5 as indicated in the following Table:

| | Mutations with respect to SEQ ID NO: 5 |
|---|---|
| 1 | G4C,Y163C |
| 2 | E183V |
| 3 | W40R |
| 4 | S311N |
| 5 | T335I |
| 6 | T48A |
| 7 | W40R,M234K |
| 8 | E65K |
| 9 | T344M |
| 10 | A72V |
| 11 | I277V |
| 12 | D346G |
| 13 | S418P |
| 14 | G4C,W40R,A72C |
| 15 | W40R,T344M |
| 16 | W46R,Q349K |
| 17 | W40R,T344M,Q349K |
| 18 | G4C,A72C,T344M |
| 19 | G4C,A72C,Q349K |
| 20 | G4C,A72C,T344M,Q349K |
| 21 | G4C,W40R,A72C,T344M |
| 22 | G4C,W40R,A72C,Q349K |
| 23 | G4C,W40R,A72C,T344M,Q349K |
| 24 | N54S,N194Y |
| 25 | V130I,H350Y |
| 26 | K92R |
| 27 | T325I |
| 28 | Y60H |
| 29 | T229A |
| 30 | D320E |
| 31 | E65V,Q349R |
| 32 | T392M |
| 33 | G4C,A72C,D346G |
| 34 | G4C,A72C,Q349R |
| 35 | G4C,A72C,D346G,Q349R |
| 36 | G4C,A72C,T344M,D346G,Q349R |
| 37 | G4C,A6G,A72C,Q349K |
| 38 | G4C,A72C,Q349K,A376T |
| 39 | G4C,A72C,Q349K,E65V,Q349R |
| 40 | G4C,A72C,Q349K,S24N |
| 41 | G4C,P12Q,A72C,Q349K,Q362H |
| 42 | N194C,Y374C |
| 43 | T243C,A375C,N194C,Y374C |
| 44 | G4C,A72C,Q349K,D320V |
| 45 | G4C,A72C,Q349K,S86T |
| 46 | G4C,A72C,D181N,Q349K |
| 47 | G4C,A72C,E183V,K304R,Q349K |
| 48 | Q1L,G4C,A72C,Q349K |
| 49 | G4C,A72C,S86T,M234V,Q349K |
| 50 | G4C,A72C,E183K,Q349K |
| 51 | G4C,A72C,S311G,Q349K |
| 52 | G4C,D64N,A72C,Q349K |
| 53 | G4C,A72C,D181 N,P224L,Q349K |
| 54 | G4C,A72C,E183K,N318Y,Q349K |
| 55 | G4C,A72C,E183V,Q349K |
| 56 | G4C,A72C,N194C,Y374C |
| 57 | G4C,A72C,N194C,Q349R,Y374C |
| 58 | N194R,Y374D |
| 59 | T243R,A375D |
| 60 | N194R,T243R,Y374D,A375D |
| 61 | Q1L,G4C,A72C,S86T,Q349R |
| 62 | Q1L,G4C,A72C,D181N,Q349R |
| 63 | Q1L,G4C,A72C,E183K,Q349R |
| 64 | Q1L,G4C,A72C,D181N,E183K,Q349R |
| 65 | Q1L,G4C,A72C,D320V,Q349R |
| 66 | Q1L,G4C,A72C,S86T,D181N,E183K,D320V,Q349R |
| 67 | G4C,A72C,Q349R,V367A |
| 68 | G4C,A72C,E183K,D202Y,N310D,Q349R |
| 69 | Q1L,G4C,A72C,Q349R |
| 70 | G4C,A72C,G251R,Q349R |
| 71 | G4C,A72C,P224L,F306Y,Q349R |
| 72 | G4C,V32G,N49S,A72C,S193L,Q349R |
| 73 | G4C,A72C,D181N,Q349R |
| 74 | G4C,A72C,D202N,Q349R |
| 75 | A72V,T335I,D346A,T393A,P436S |
| 76 | A72V,T3351,D346A,T393A |
| 77 | W40R,D346A,T393A |
| 78 | A72V,D346A,T393A |
| 79 | G4C,E65V,A72C,Q349R |
| 80 | G4C,A72C,D202G,D320N,Q349R,A358E |
| 81 | G4C,A72C,D320V,Q349R |
| 82 | G4C,E65V,A72C,Y244H,Q349R |
| 83 | G4C,A72C,E183K,Q349R |
| 84 | G4C,A72C,E183K,D346E,Q349R |
| 85 | G4C,A72C,S311G,Q349R |
| 86 | G4C,A72C,P224L,Q349R |
| 87 | G4C,A72C,S236Y,Q349R |
| 88 | A72V,D320V,D346A |
| 89 | A72V,D346A |
| 90 | W40R,T3351,D346A,T393A |
| 91 | G4C,A72C,D320V,Q349K |
| 92 | G4C,A72C,S311G,D320V,Q349K |
| 93 | Q1L,G4C,A72C,Q349K,T392M |
| 94 | D320V,Q349K |
| 95 | G4C,E172C,D202V,D320V,Q349K |
| 96 | G4C,A72C,D320V,D346V,Q349K |
| 97 | Q1L,G4C,A72C,D202N,Q349K |
| 98 | Q1L,G4C,A68T,A72C,Q349K |
| 99 | Q1L,G4C,A72C,K154R,Q349K,T393I |
| 100 | Q1L,G4C,E65K,A72C,Q349K |
| 101 | Q1L,G4C,A72C,D181N,D247N,Q349K |
| 102 | Q1L,G4C,Q28R,A72C,Q349K |
| 103 | Q1L,G4C,A72C,A145T,H203R,Q349K,T403K ⁻ |
| 104 | Q1L,G4C,D64N,A72C,Q349K |
| 105 | Q1L,G4C,A72C,V152A,Q349K |
| 106 | Q1L,G4C,T15S,A72C,Y244F,Q349K |
| 107 | Q1L,G4C,A6V,A72C,Q349K |
| 108 | Q1L,G4C,A72C,Y155C,Q349K |
| 109 | C11L,G4C,A72C,S311N,Q349K |
| 110 | Q1L,G4C,A72C,D346V,Q349K |
| 111 | Q1L,G4C,A72C,Q349K,T392K |
| 112 | Q1L,G4C,A72C,S311G,Q349K |
| 113 | G4C,A72C,N194K,T243Y,Q349R,A375N |
| 114 | G4C,A72C,N194D,T243A,Q349R,Y374P,A375Y |
| 115 | G4C,A72C,N1941,T243Y,Q349R,Y374P,A375R |
| 116 | G4C,A72C,N194K,Q349R,Y374P,A375Q |
| 117 | G4C,A72C,N194L,T243Y,Q349R,Y374R,A375L |
| 118 | G4C,A72C,N194G,T243F,Q349R,Y374P,A375R |
| 119 | G4C,A72C,N194L,T243Q,Q349R,Y374P,A375V |
| 120 | G4C,A72C,N194K,T243P,Q349R,Y374H,A375E |
| 121 | G4C,A72C,N1941,T243D,Q349R,Y374P,A375Y |
| 122 | G4C,A72C,N194Q,T243V,Q349R,Y374P,A375Y |
| 123 | G4C,A72C,N194S,T243W,Q349R,Y374S,A375G |
| 124 | G4C,A72C,N194Y,T243L,Q349R,Y374S,A375R |
| 125 | G4C,A72C,N194V,T243M,Q349R,Y374A,A375T |
| 126 | G4C,A72C,T243G,Q349R,Y374P,A375M |
| 127 | G4C,A72C,T243Q,Q349R,Y374P,A375M |
| 128 | G4C,A72C,N194Y,T243V,Q349R,Y374P |

Furthermore, the present application discloses a polypeptide having cellobiohydrolase activity comprising an amino acid sequence having at least 85%, preferably at least 95%, more preferably at least 98%, even more preferably at least 99%, and most preferably 100% sequence identity to SEQ ID NO:12 wherein one or more of the following amino acid residues of the sequence defined by SEQ ID NO: 12 are modified by substitution or deletion: Q1, T15, Q28, W40, C72, V133, V155, Y158, T162, Y247, N307, G308, E317, S341, D345, Y370, T389, Q406, N441, R442, T452, S456, P458, G459, H464 and/or V478.

In a preferred embodiment, the polypeptide having cellobiohydrolase activity comprising an amino acid sequence having at least 85 % sequence identity to SEQ ID NO: 12 comprises one or more of the following modified amino acid residues of the sequence defined by SEQ ID NO: 12:

| | Exchange with respect to SEQ ID NO: 12 |
|---|---|
| 1 | Q1L |
| 2 | T15S |
| 3 | Q28R |
| 4 | W40R |
| 5 | C72V |
| 6 | V133I |
| 7 | V155A,E |
| 8 | Y158C |
| 9 | T162E |
| 10 | Y247F,H |
| 11 | N307D |
| 12 | G308N |
| 13 | E317V,N |
| 14 | S341M |
| 15 | D345R,K |
| 16 | Y370P,R,H,S,A |
| 17 | T389A |
| 18 | Q406G |
| 19 | N441D |
| 20 | R442S,G |
| 21 | T452A |
| 22 | S456L,P |
| 23 | P458L,del |
| 24 | G459D |
| 25 | H464L,Q,R |
| 26 | V478A,I |

Another aspect of the disclosure relates to the application of the isolated polypeptides and variants thereof of the present invention for the complete or partial hydrolysis of cellulosic material. The cellulosic material can be of natural, processed or artificial nature. "Cellulosic material" herein shall be defined as all sorts of pure, non-pure, mixed, blended or otherwise composed material containing at least a fraction of β-1-4-linked D-glucosyl polymers of at least 7 consecutive subunits. Prominent examples of cellulosic materials are all sort of cellulose containing plant materials like wood (soft and hard), straw, grains, elephant grass, hey, leaves, cotton and materials processed there from or waste streams derived from such processes. Cellulosic material used in an enzymatic reaction is herein also referred to as cellulosic substrate.

The hydrolysis of the cellulose material can be a sequential process following cellobiohydrolase production or contemporary to the production in the yeast cell (consolidated bioprocess). The expression of cellulolytic enzymes in yeast is of special interest due to the ability of many yeasts to ferment the released sugars (C6 or C5) to ethanol or other metabolites of interest.

A further aspect of the application thus relates to the application of whole cells expressing the polypeptide or variant thereof according to the present invention for the processing of cellulosic materials.

In a particular aspect, the present application discloses the use of a polypeptide and variants thereof or the composition of the present invention for the enzymatic degradation of cellulosic material, preferably lignocellulosic biomass, and/or for textiles processing and/or as ingredient in detergents and/or as ingredient in food or feed compositions.

### Examples

### Example 1: Preparation of Pichia pastoris expression plasmid

Expression plasmids for the constitutive expression of protein from transformed *Pichia pastoris* hosts are prepared by assembly of an expression cassette consisting of a *Pichia pastoris* gyceraldehyde phosphate dehydrogenase (GAP) promoter, a *Saccharomyces cerevisiae* SPα (mating factor alpha signal peptide), a multiple cloning site (MCS) and the 3'-GAP-terminator sequence. For selection purposes a kanamycine resistance gene is used under control of the EM7 or TEF promoter for bacterial or yeast selection purposes, respectively. The resulting plasmid vectors are designated as pV1 (Figure 1) and pV2 (alternative MCS) Transformation and expression cultivation are done essentially as described by Waterham, H. R., Digan, M. E., Koutz, P. J., Lair, S. V., Cregg, J. M. (1997). Isolation of the Pichia pastoris glyceraldehyde-3-phosphate dehydrogenase gene and regulation and use of its promoter. Gene, 186, 37-44 and Cregg, J.M.: Pichia Protocols in Methods in Molecular Biology, Second Edition, Humana Press, Totowa New Jersey 2007.

### Comparative example 1: Construction of Pichia pastoris expression constructs for CBHI sequences

CBHI genes of *Trichoderma viride* (CBH-f), *Humicola grisea* (CBH-d), *Thermoascus aurantiacus* (CBH-e), *Talaromyces emersonii* (CBH-b), and fusions of the cellulose binding domain of *Trichoderma reesei* CBHI with the *Talaromyces emersonii* CBHI (CBH-a) or the *Humicola grisea* CBHI (CBH-g) are amplified using the oligo nucleotide pairs and templates (obtained by gene synthesis) as given in the table. The fusion gene encoding SEQ ID NO: 2 is generated by overlap extension PCR using the PCR-Fragments generated from SEQ ID NOs:5 and 11. Phusion DNA polymerase (Finnzymes) is used for the amplification PCR.

**Table 1: Primers and templates for the amplification of CBH-a, CBH-b, CBH-d, CBH-e, CBH-f and CBH-g**

| | Fragment | Primer forward | Primer reverse | Template |
|---|---|---|---|---|
| CBH-f | Trichoderma viride CBHI | | | SEQID NO. 13 |
| CBH-d | Humicula grisea CBHI | | | SEQID NO. 7 |
| CBH-e | Thermoascus aurantiacus CBHI | | | SEQID NO. 9 |
| CBH-b | Talaromyces emersonii CBHI | | | SEQID NO. 5 |
| CBH-a part1 | Talaromyces emersonii CBH fusion fragment part1 | | | SEQID NO. 5 |
| CBH-a part2 | Trichoderma reesei CBHI binding domain fusion fragment part2 | | | SEQID NO.11 |
| CBH-a | Talaromyces emersonii CBHI fusion protein | | | 5a+5b SEQID NO. 2 |
| CBH-g part1 | Humicola grisea CBHI fusion fragment part1 | | | SEQID NO. 19 |
| CBH-g part2 | Trichoderma reesei CBHI binding domain fusion fragment part2 | | | SEQID NO. 11 |
| CBH-g | Humicula grisea fusion protein | | | 6a+6b SEQID NO. 15 |

PCR fragments of expected length are purified from agarose gels after electrophoresis using the Promega® SV PCR and Gel Purification kit. Concentration of DNA fragments are measured on a Spectrophotometer and 0,2pmol of fragments are treated with 9U of T4-DNA polymerase in the presence of 2,5mM dATP for 37,5 min at 22,5°C and treated fragments are annealed with T4-DNA-Polymerase/dTTP treated *Sma*I-linearized pV1 plasmid DNA and afterwards transformed into chemically competent Escherichia coli Top10 cells. Deviant from the described procedure the product generated by the primer pair according to the table lane 11 encoding the *Humicula grisea* fusion protein fragments are cloned via the introduced *Sph*I and *Sal*I site to pV2. Transformants are controlled by sequencing of isolated plasmid DNA.

### Comparative example 2: Expression of CBHI Genes in Pichia pastoris

Plasmids of Example 2 are transformed to electro-competent Pichia pastoris CBS 7435 cells and transformants are used to inoculate cultures in YPD medium containing 200mg/l, which are incubated for 5 days at 27°C in a rotary shaker at 250 rpm. Culture supernatants were separated by centrifugation at 5000xg for 30 minutes in a Sorvall Avant centrifuge. Supernatants were concentrated on spin columns with cut-off size of 10kDa. Protein pattern of such concentrated supernatants were analyzed by SDS-PAGE (Laemmli et al.) and gels were stained with colloidal Commassie blue stain. Enzymatic activity was determined by incubation of the supernatant with 2mM solutions of p-nitrophenyl-β-D-lactoside or 200µM solutions of 4-methyl-umbelliferyl-β-D-lactoside in 50 mM sodium acetate buffer (pH 5) for 1 hour. The reaction was stopped my addition of equal volumes of 1 M sodium carbonate solution and determination of released p-nitrophenol or 4-methyl umbelliferone by measurement of the absorbance at 405 nm or the fluorescence at 360 nm/ 450 nm excitation/emission.

### Comparative example 3: Genome integration and expression of the Talaromyces emersonii CBHI-T. reesei CBHII-CBD fusion sequence in Pichia pastoris

The DNA-fragment of the fusion gene are generated by 2 step overlap extension PCR using the oligo nucleotide pairs and synthetic templates as indicated in the table (of Example 2). T4-DNA polymerase treated full length fragment was annealed with the linear pV3 vector fragment by slowly reducing the temperature from 75°C to 4°C. The pV3 plasmid contains a fusion of the mating factor alpha signal peptide to a multiple cloning site, situated downstream the of a *Pichia pastoris* AOXI promoter. Transformation of the annealed solution into chemical competent E. coli cells yields transformants, which are selected by their Teocine resistance checked for containing expected construct plasmid by restriction analysis and sequencing. pV3-CBH-a plasmid preparations are linearized with SacI and approximately 1 µg of linear DNA-fragments are transformed to Pichia pastoris electrocompetent cells. 94 Transformants from YPD-Zeocin plates are afterwards checked for expression by cultivation in 500µl 96-deepwell Plate cultures in BMMY-medium containing 1% methanol and 0.5 % methanol was fed every 24h for 5 days (350 rpm/27°C; humidified orbital shaker with 2,5 cm amplitude. Supernatants are tested for activity on 4-MUL and clones with highest expression levels are selected and again evaluated under same conditions.

For fermentation in an Infors Multifors bioreactor the strain producing the highest enzyme concentration is selected. A YPD-Zeocin (100g/l) pre-culture is chosen for inoculation of Mineral medium consisting of phosphate-buffer, magnesium sulphate and chloride, trace elements/biotin and glycerol, with pH calibration using ammonia and phosphoric acid. After metabolism of the batch glycerol (2%) additional glycerol feed is maintained for 1 day before the feed is changed to methanol to shift to inductive conditions for the AOXI promoter. Under these conditions the fermentation is kept for 5 days. Cells are separated from the fermentation liquid by centrifugation at 5000xg for 30 minutes. Supernatants are analyzed for total Protein using Bradford Reagent and BSA Standards (Biorad). SDS-PAGE / Coomassie Brilliant blue staining is used to analyze the Protein Pattern on the SDS-PAGE.

### Example 2: Trichoderma reesei expression vector construct

SbfI/SwaI digested pSCMB100 plasmid DNA was transformed into *Trichoderma reesei* SCF41 essentially as described by Penttilä et al 1997. 10µg of linear DNA was used for the transformation of 10⁷ protoplasts. Selection of transformants was done by growth of the protoplasts on Mandel's Andreotti media plates with overlay agar, containing hygromycine as selective agent (100mg/l). Transformants were further purified by passage over sporolation media plates and re-selection of spores on hygromycin media. From re-grown mycelia genomic DNA was isolated and the replacement event verified by PCR. Transformants verified in being true replacement strains were further tested for secretion of recombinant protein.

### Example 3: Expression of Talaromyces emersonii CBHI / Trichoderma reesei -CBD fusion (CBH-ah) from Trichoderma reesei

Expression of recombinant CBHI replacement strains of *Talaromyces emersonii* CBHI / *Trichoderma reesei* -CBD fusion with 6x His-Tag in *Trichoderma reesei* Q6A (ATCC 13631) was done in shakeflask cultures containing 40ml Mineral medium containing 2% Avicel in 300ml flasks and cultivation at 30°C/250rpm for 6 days. Supernatants recovered by centrifugation and further analyzed by SDS-PAGE and Bradford Protein assays.

### Example 4: Screening thermo stability variants

Random mutagenesis libraries of the *Talaromyces emersonii* CBHI / *Trichoderma reesei -* CBD fusion (with 6x His-Tag) gene were generated using error prone PCR applying manganese containing bufferers and inbalanced dNTP concentrations in the *Taq*-DNA polymerase reaction micture, used for PCR-amplification, essentially as described by Craig and Joyce (R.Craig Cadwell and G.F. Joyce, 1995. Mutagenic PCR, in PCR Primer: a laboratory manual, ed. C. W. Dieffenbach and G. S. Dveksler, Cold Spring Harbor Press, Cold Spring Harbor, ME, 583-589). As template the wild type fusion gene (SEQ ID NO: 17) or mutants thereof were chosen. Mutated PCR-Fragments were cloned to the pPKGMe Plasmid using Sphl and *Hind*III endonucleases and T4-DNA-ligase.

Libraries of the *Talaromyces emersonii* CBHI / *Trichoderma reesei* -CBD fusion (with 6x His-Tag) gene variants were distributed in 1536 well plates with well occupation number close to 1. Enzyme was expressed over 7 days in a volume of 4µl YPG-G418 medium. For evaluation of the properties of the variants 2µl samples of culture supernatants were transferred to plates containing a suspension of milled straw, acetate buffer and beta-glucosidase. After incubation of the sealed reaction plates for 48 hours at defined temperatures the glucose concentration was determined using Amplex red in the presence of GOX and HRP by analyzing the fluorescence level. Best-performing Hits were re-cultivated and re-evaluated. Plasmids of confirmed CBH-ah variants were recovered (Pierce DNAzol Yeast genomic DNA Kit) and sequenced using oligonucleotides alpha-f (5' TACTATTGCCAGCATTGCTGC-3') and oli740 (5'-TCAGCTATTTCACATACAAATCG-3').

### Example 5: Determination of Substrate Conversion Capacity at different temperatures for indication of the thermostability of CBH-ah-Variants using 4-methylumbellifery-β-D-lactoside (4-MUL)

For precise comparison of the thermal stability culture supernatants containing the secreted cellobiohydrolase variants were diluted tenfold in sodium acetate buffer (50mM, pH 5) and 10µl samples were incubated with 90µl of 200µM 4-MUL (in buffer) in the temperature gradient of an Eppendorff Gradient Thermocycler. A temperature gradient of 20°C reaching from 55°C to 75°C was applied to 12 reaction mixtures for for each sample for one hour. The temperature profile could be recorded after addition of 100µl 1 M sodium carbonate solution to each reaction and measurement of the fluorescence intensity at 360nm/454nm in a Tecan Infinite M200 plate reader. For comparison of the thermostability the values were normalized between 1 and 0 for the maximum and minimum fluorescence count (Figure 7).

**Table 2: Listing of Mutants of SEQ ID NO: 18 with improved IT50 values.**

| | Mutations with respect to SEQ ID NO: 18 | IT50 [°C] |
|---|---|---|
| | none | 59,8 |
| 1 | N194R,Y374D | 59,8 |
| 2 | S467T | 60,1 |
| 3 | G4C,A72C,Q349K,S24N | 60,1 |
| 4 | T392M | 60,2 |
| 5 | W40R,D320V,T393A,N445D | 60,2 |
| 6 | W40R | 60,3 |
| 7 | R446G | 60,6 |
| 8 | D346G,R453G | 60,9 |
| 9 | Y496F | 60,9 |
| 10 | T3351,D346A,T393A,D410G | 61,0 |
| 11 | T2431,T325A,V482A | 61,0 |
| 12 | N194R,T243R,Y374D,A375D | 61,1 |
| 13 | E65K | 61,1 |
| 14 | T48A | 61,2 |
| 15 | G4C,A72C,T344M | 61,2 |
| 16 | T243R,A375D | 61,3 |
| 17 | W40R,K159E,N445D,A501S | 61,3 |
| 18 | T344M | 61,3 |
| 19 | W40R,M234K | 61,3 |
| 20 | Q349R,T393A,P436S,N445D | 61,4 |
| 21 | Q349R,A354T,D373E,N445D | 61,4 |
| 22 | G4C,W40R,A72C,T344M | 61,4 |
| 23 | G4C,A72C,N194L,T243Y,Q349R,Y374R,A375L | 61,5 |
| 24 | G4C,D64N,A72C,Q349K | 61,5 |
| 25 | W40R,T344M | 61,5 |
| 26 | W40R,D346A,T393A | 61,6 |
| 27 | N158D,G486S,Y495C | 61,6 |
| 28 | D320E | 61,6 |
| 29 | A72V | 61,6 |
| 30 | E183V | 61,6 |
| 31 | Q349R,N445D | 61,6 |
| 32 | W40R,C489R | 61,7 |
| 33 | W40R,Q349K | 61,9 |
| 34 | G4C,A72C,Q349K,E65V,Q349R | 61,9 |
| 35 | S311N | 62,0 |
| 36 | D320V,D346A,Q349R,T393A,N445D | 62,1 |
| 37 | T335I | 62,2 |
| 38 | A72V,D346A | 62,2 |
| 39 | D320V,D346A,T393A,N445D | 62,2 |
| 40 | G4C,A72C,N194S,T243W,Q349R,Y374S,A375G | 62,3 |
| 41 | E65V,Q349R | 62,3 |
| 42 | A72V,T3351,D346A,N445D | 62,3 |
| 43 | G4C,A72C,N194L,T243,Q,Q349R,Y374P,A375V | 62,3 |
| 44 | G4C,A72C,D346G | 62,3 |
| 45 | W40R,T344M,Q349K | 62,3 |
| 46 | G4C,Y163C | 62,4 |
| 47 | G4C,V32G,N49S,A72C,S193L,Q349R | 62,4 |
| 48 | G4C,A72C,D346G,Q349R | 62,6 |
| 49 | W40R,D320V,Q349K,P436S,N445D | 62,7 |
| 50 | G4G,A72C,D181N,Q349R | 62,7 |
| 51 | G4C,A72C,Q349K | 62,7 |
| 52 | A72V,Q349R,N445D | 62,8 |
| 53 | G4C,A72C,T344M,Q349K | 62,8 |
| 54 | G4C,A72C,D320V,Q349K | 62,8 |
| 55 | G4C,A72C,P224L,F306Y,Q349R | 62,9 |
| 56 | A72V,T335I,D346A,T393A,N445D | 62,9 |
| 57 | G4C,A72C,T344M,D346G,Q349R | 63,0 |
| 58 | A72V,D346A,T393A | 63,0 |
| 59 | G4C,A72C,Q349R,R446S,T456A | 63,0 |
| 60 | G4C,W40R,A72C,T344M,Q349K | 63,0 |
| 61 | A72V,D320V,D346A | 63,1 |
| 62 | G4C,A72C,N194Y,T243L,Q349R,Y374S,A375R | 63,1 |
| 63 | G4C,A72C,Q349K,T448A,T449A | 63,2 |
| 64 | G4C,E65V,A72C,Y244H,Q349R | 63,3 |
| 65 | G4C,A72C | 63,3 |
| 66 | G4C,A72C,P224L,Q349R,S409L,P429S | 63,4 |
| 67 | G4C,A72C,D202G,D320N,Q349R,A358E | 63,4 |
| 68 | G4C,A72C,D320V,Q349R,H508R | 63,4 |
| 69 | G4C,A72C,Q349K,S86T | 63,4 |
| 70 | A72V,T335I,D346A,T393A,P436S | 63,4 |
| 71 | G4C,A72C,E183V,K304R,Q349K | 63,5 |
| 72 | G4C,A72C,T243G,G3349R,Y374P,A375M | 63,5 |
| 73 | G4C,A72C,Q349R,T465I | 63,6 |
| 74 | G4C,A72C,N194V,T243M,Q349R,Y374A,A375T | 63,6 |
| 75 | G4C,D64N,A72C.Q349R,A358E,P464Q | 63,6 |
| 76 | G4C,A72C,Q349K,Q28R,S193T,Q490L | 63,6 |
| 77 | G4C,A72C,E183K,Q349K | 63,6 |
| 78 | G4C,A72C,S311N,Q349K,A455T,H509Q | 63,6 |
| 79 | G4C,A72C,N194K,Q349R,Y374P,A375Q | 63,6 |
| 80 | G4C,A72C,D181N,Q349K | 63,6 |
| 81 | W40R,D320V,Q349K,T393A,N445D | 63,7 |
| 82 | W40R,T3351,D346A,T393A | 63,7 |
| 83 | G4C,A72C,N194K,T243P,Q349R,Y374H,A375E | 63,7 |
| 84 | G4C,A72V,Q349R,P462del | 63,8 |
| 85 | G4C,A72C,S236Y,Q349R | 63,8 |
| 86 | G4C,A72C,S311G,Q349K | 63,8 |
| 87 | A72V,D320V,T335I,D346A,T393A,N445D | 63,8 |
| 88 | G4C,A72C,S86T,M234V,Q349K | 63,8 |
| 89 | G4C,A72C,G251R,Q349R | 63,9 |
| 90 | QIL,G4C,A68T,A72C,Q349K,H505N | 63,9 |
| 91 | A72V,T335I,D346A,T393A | 63,9 |
| 92 | G4C,A72C,E183K,Q349R | 63,9 |
| 93 | G4C,A72C,Q349R | 63,9 |
| 94 | Q1L,G4C,A72C,H203R,Q349K,P442S | 63,9 |
| 95 | G4C,A72C,Q349K,G434S,G470D | 64,0 |
| 96 | G4C,W40R,A72C,Q349K | 64,0 |
| 97 | G4C,A72C,Q349R,V367A | 64,0 |
| 98 | G4C,A72C,S311G,D320V,Q349K | 64,0 |
| 99 | W40R,T335I,D346A,T393A,P436S | 64,0 |
| 100 | A72V,D346A,T393A,N445D | 64,1 |
| 101 | Q1L,G4C,A72C,D202N,Q349K | 64,1 |
| 102 | G4C,A72C,D320V,Q349R | 64,1 |
| 103 | Q1L,G4C,A72C,K154R,G1349K,T393I | 64,1 |
| 104 | G4C,A72C,N194G,T243F,Q349R,Y374P,A375R | 64,1 |
| 105 | A72V,D320V,D346A,T393A,N445D | 64,1 |
| 106 | G4C,A72C,E183V,Q349K | 64,1 |
| 107 | G4C,A72C,E183K,N318Y,Q349K | 64,1 |
| 108 | G4C,A72C,K92R,Q349K,N493D | 64,1 |
| 109 | Q1L,G4C,A72C,Q349K | 64,2 |
| 110 | M234I,G438deI | 64,2 |
| 111 | G4C,A72C,Q349R,G459D | 64,2 |
| 112 | G4C,A72C,Q349R,Y422F | 64,2 |
| 113 | G4C,T48A,A72C,Q349R,P480S | 64,2 |
| 114 | E187K,D320V,P442del | 64,2 |
| 115 | G4C,S24N,E65K,A72C,Q349R,I430L,G439D | 64,2 |
| 116 | A72V,D320V,T335I,D346A,T393A,P436S | 64,3 |
| 117 | Q1L,G4C,A72C,S193P,Q349K,V482I | 64,3 |
| 118 | G4C,A72C,D320V,Q349K,G443D,L492Q | 64,3 |
| 119 | G4C,A72C,Q349K,D320V | 64,4 |
| 120 | G4C,A72C,N194K,T243Y,Q349R,A375N | 64,4 |
| 121 | Q1L,G4C,Q28R,A72C,Q349K,H468L | 64,5 |
| 122 | G4C,E65V,A72C,Q349R | 64,5 |
| 123 | D320V,Q349K | 64,5 |
| 124 | G4C,A72C,D181N,P224L,Q349K | 64,5 |
| 125 | G4C,A72C,T243Q,Q349R,Y374P,A375M | 64,5 |
| 126 | Q1L,G4C,A72C,D320V,Q349R | 64,5 |
| 127 | Q1L,G4C,T15S,A72C,Y244F,Q349K | 64,5 |
| 128 | G4C,A72C,E183K,D346E,Q349R | 64,6 |
| 129 | Q1L,G4C,A72C,Q349K,T392M | 64,6 |
| 130 | G4C,A72C,D202N,S311N,Q349R,N493D | 64,6 |
| 131 | G4C,A72C,N194D,T243A,Q349R,Y374P,A375Y | 64,6 |
| 132 | G4C,A72C,N194Y,T243V,Q349R,Y374P | 64,7 |
| 133 | Q1L,G4C,A72C,Q349R | 64,7 |
| 134 | G4C,A72C,D202N,Q349R,H507Y | 64,7 |
| 135 | G4C,A72C,P224L,Q349R,A503V | 64,7 |
| 136 | N194C,Y374C | 64,7 |
| 137 | Q1L,G4C,A72C,D181N,Q349R | 64,8 |
| 138 | G4C,A72C,D320V,D346V,Q349K | 64,8 |
| 139 | Q1L,G4C,A72C,V152A,Q349K | 64,8 |
| 140 | G4C,A72C,E183K,D202Y,N310D,Q349R | 64,8 |
| 141 | Q1L,G4C,Q28R,A72C,Q349K | 64,8 |
| 142 | Q1L,G4C,A72C,Q349K,Y422F | 64,8 |
| 143 | G4C,A72C,D202V,D320V,Q349K,H504Y | 64,8 |
| 144 | Q1L,G4C,A72C,D181N,D247N,Q349K,S502T | 64,8 |
| 145 | Q1L,G4C,A68T,A72C,Q349K,G439D,R453S | 64,9 |
| 146 | Q1L,G4C,A72C,Q349K,H468R | 64,9 |
| 147 | Q1L,G4C,D64N,A72C,G1349K | 64,9 |
| 148 | G4C,A72C,E183K,Q349R,P464L | 64,9 |
| 149 | G4C,A72C,N1941,T243Y,Q349R,Y374P,A375R | 65,0 |
| 150 | Q1L,G4C,A72C,Q349K,H508L | 65,0 |
| 151 | Q1L,G4C,A72C,E183K,Q349R | 65,0 |
| 152 | G4C,A72C,S311G,Q349R | 65,0 |
| 153 | Q1L,G4C,A72C,S311N,Q349K,G463D | 65,1 |
| 154 | Q1L,G4C,A72C,S86T,Q349R | 65,1 |
| 155 | Q1L,G4C,E65K,A72C,Q349K | 65,2 |
| 156 | Q1L,G4C,A72C,D181N,Q349K,T451S | 65,2 |
| 157 | Q1L,G4C,A6V,A72C,Q349K | 65,3 |
| 158 | G4C,A72C,N1941,T243D,Q349R,Y374P,A375Y | 65,3 |
| 159 | G4C,A72C,N194C,Y374C | 65,6 |
| 160 | Q1L,G4C,A72C,A145T,H203R,Q349K,T403K | 65,8 |
| 161 | Q1L,G4C,A72C,S311G,Q349K | 66,0 |
| 162 | Q1L,G4C,A72C,D202N,Q349K,G486D | 66,0 |
| 163 | Q1L,G4C,A72C,I200F,Q349K,L500I | 66,0 |
| 164 | Q1L,G4C,A68T,A72C,Q349K | 66,1 |
| 165 | Q1L,G4C,A72C,D346V,Q349K | 66,2 |
| 166 | G4C,A72C,N194Q,T243V,Q349R,Y374P,A375Y | 66,3 |
| 167 | Q1L,G4C,A72C,Q349K,T392K | 66,3 |
| 168 | Q1L,G4C,A72C,D181N,E183K,Q349R | 66,4 |
| 169 | Q1L,G4C,A72C,S311N,Q349K | 66,5 |
| 170 | G4C,A72C,N194C,Q349R,Y374C | 66,5 |
| 171 | Q1L,G4C,A72C,Y155C,Q349K,A503T | 67,0 |
| 172 | Q1L,G4C,A72C,S86T,D181N,E183K,D320V,Q349R | 67,0 |

### Example 6: Characterization of Variants of the Talaromyces emersonii CBHI/Trichoderma reesei-CBD fusion (with 6x His-Tag)

80 mL of fermentation broth were concentrated to a final volume of approx. 1mL. After determination of protein concentration (Bradford reagent, Biorad, Germany, Standard is BSA form Sigma-Aldrich, Germany) 1.2mg of protein were purified with the Ni-NTA Spin kit (Qiagen, Germany). The purified CBH1 fraction was subsequently assayed by performing a hydrolysis reaction on pretreated (acid pretreatment) wheat straw. 12,5mg (dry mass) of pretreated wheat straw is mixed with 0,0125mg of purified CBH1 and 40CBU Novo188 (Novozymes, Denmark) per mg of CBH1. 50mM sodium acetate (Sigma-Aldrich, Germany) is added up to 500µL. The assay is kept at temperatures ranging from 50°C to 65°C for 48 hours and analysed by HPLC to determine the temperature dependent glucose content.

## Claims

1. A polypeptide having cellobiohydrolase activity, wherein the polypeptide comprises an amino acid sequence having at least 85 % sequence identity to SEQ ID NO: 2, wherein the amino acid residue at position Q1 of SEQ ID NO: 2 is modified by substitution or deletion.

2. The polypeptide according to claim 1, wherein the polypeptide maintains 50 % of its maximum substrate conversion capacity when the conversion is done for 60 minutes at a temperature of 60°C or higher.

3. The polypeptide according to claim 1 or 2, wherein the polypeptide comprises an amino acid sequence having at least 90 %, preferably at least 95 %, more preferably at least 99 % sequence identity to SEQ ID NO: 2.

4. The polypeptide according to one or more of the preceding claims, wherein the amino acid sequence of the polypeptide has the sequence as defined by SEQ ID NO: 2, or a sequence as defined by SEQ ID NO: 2 wherein 1 to 75 amino acid residues, more preferably 1 to 35 amino acid residues are substituted, deleted, or inserted.

5. The polypeptide according to claim 4, wherein additionally one or more of the following amino acid residues of the sequence defined by SEQ ID NO: 2 are modified by substitution or deletion: positions G4, A6, T15, Q28, W40, D64, E65, A72, S86, K92, V130, V152, Y155, K159, D181, E183, N194, D202, P224, T243, Y244, I277, K304, N310, S311, N318, D320, T335, T344, D346, Q349, A358, Y374, A375, T392, T393, D410, Y422, P442, N445, R446, T456, S460, P462, G463, H468 and/or V482 of amino acids 1 to 500 of SEQ ID NO: 2.

6. The polypeptide according to claim 5, wherein the polypeptide comprises one or more of the following preferred exchanges with respect to the sequence defined by SEQ ID NO: 2:
| Position | Preferred Exchange |
|---|---|
| Q1 | L |
| G4 | C |
| A6 | G,V |
| T15 | S |
| Q28 | Q,R |
| W40 | R |
| D64 | N |
| E65 | K,V |
| A72 | C,V |
| S86 | T |
| K92 | K,R |
| V130 | I,V |
| V152 | A,E |
| Y155 | C |
| K159 | E |
| D181 | N |
| E183 | V,K |
| N194 | C,R,Y,D,K,I,L,G,Q,S,V |
| D202 | Y,N,G |
| P224 | L |
| T243 | I,C,R,Y,A,F,Q,P,D,V,W,L,M |
| Y244 | F,H |
| I277 | V |
| K304 | R |
| N310 | D |
| S311 | G,N |
| N318 | Y |
| D320 | V,E,N |
| T335 | I |
| T344 | M |
| D346 | G,A,E,V |
| Q349 | R,K |
| A358 | E |
| Y374 | C,P,R,H,S,A |
| A375 | C,D,N,Y,R,Q,L,V,E,G,T,M |
| T392 | C,D,K |
| T393 | A |
| D410 | G |
| Y422 | F |
| P442 | S,del |
| N445 | D |
| R446 | S,G |
| T456 | T,A |
| S460 | L,P |
| P462 | L,del |
| G463 | D |
| H468 | L,Q,R |
| V482 | A,I |

7. The polypeptide according to one or more of the preceding claims, wherein the polypeptide has an amino acid sequence selected from the list of the following mutations of SEQ ID NO: 2:
| | |
|---|---|
| 75 | Q1L,G4C,A72C,Q349K |
| 88 | Q1L,G4C,A72C,S86T,Q349R |
| 89 | Q1L,G4C,A72C,D181N,Q349R |
| 90 | Q1L,G4C,A72C,E183K,Q349R |
| 91 | Q1L,G4C,A72C,D181N,E183K,Q349R |
| 92 | Q1L,G4C,A72C,D320V,Q349R |
| 93 | Q1L,G4C,A72C,S86T,D181N,E183K,D320V,Q349R |
| 98 | Q1L,G4C,A72C,Q349R |
| 148 | Q1L,G4C,A72C,Q349K,T392M |
| 153 | Q1L,G4C,A68T,A72C,Q349K,G439D,R453S |
| 154 | Q1L,G4C,A72C,D202N,Q349K |
| 155 | Q1L,G4C,A68T,A72C,Q349K |
| 156 | Q1L,G4C,A72C,K154R,Q349K,T393I |
| 157 | Q1L,G4C,A72C,S193P,Q349K,V482I |
| 158 | Q1L,G4C,A72C,H203R,Q349K,P442S |
| 159 | Q1L,G4C,A72C,Q349K,H468R |
| 160 | Q1L,G4C,A72C,D202N,Q349K,G486D |
| 161 | Q1L,G4C,E65K,A72C,Q349K |
| 162 | Q1L,G4C,A72C,Q349K,Y422F |
| 163 | Q1L,G4C,Q28R,A72C,Q349K,H468L |
| 164 | Q1L,G4C,A72C,D181N,D247N,Q349K |
| 165 | Q1L,G4C,A72C,D181N,Q349K,T451S |
| 166 | Q1L,G4C,Q28R,A72C,Q349K |
| 167 | Q1L,G4C,A72C,A145T,H203R,Q349K,T403K |
| 168 | Q1L,G4C,A72C,1200F,Q349K,L500I |
| 169 | Q1L,G4C,D64N,A72C,Q349K |
| 170 | Q1L,G4C,A72C,V152A,Q349K |
| 171 | Q1L,G4C,T15S,A72C,Y244F,Q349K |
| 172 | Q1L,G4C,A6V,A72C,Q349K |
| 173 | Q1L,G4C,A72C,S311N,Q349K,G463D |
| 174 | Q1L,G4C,A72C,Y155C,Q349K |
| 175 | Q1L,G4C,A72C,S311N,Q349K |
| 176 | Q1L,G4C,A72C,D346V,Q349K |
| 177 | Q1L,G4C,A72C,Q349K,T392K |
| 178 | Q1L,G4C,A72C,S311G,Q349K |
| 179 | Q1L,G4C,A72C,S311G,Q349K,H468R |

8. The polypeptide according to one or more of the preceding claims, which is expressed and secreted at a level of more than 100 mg/l, more preferably of more than 200 mg/l, particularly preferably of more than 500 mg/l, and most preferably of more than 1 g/l into the supernatant after introduction of a nucleic acid encoding a polypeptide having an amino acid sequence with at least 85% sequence identity to the SEQ ID NO: 2 into a yeast, wherein the amino acid residue at position Q1 of SEQ ID NO 2 must be modified by substitution or deletion.

9. A nucleic acid encoding the polypeptide of one or more of claim 1 to 8, preferably having at least 95% identity to SEQ lD NO: 1.

10. A vector comprising the nucleic acid of claim 9.

11. A host cell transformed with a vector of claim 10.

12. The host cell of claim 11, wherein the host cell is derived from the group consisting of Saccharomyces, Schizosaccharomyces, Kluyveromyces, Pichia, Pichia, Hansenula, Aspergillus, Trichoderma, Penicillium, Candida and Yarrowina.

13. Composition comprising the polypeptide of one or more of claims 1 to 9 and one or more endoglucanases and/or one or more beta-glucosidases and/or one or more further cellobiohydrolases and/or one or more xylanases.

14. Use of the polypeptide according to one or more of claims 1 to 9 or of the composition of claim 13 for the enzymatic degradation of lignocellulosic biomass, and/or for textiles processing and/or as ingredient in detergents and/or as ingredient in food or feed compositions.

## Patentansprüche

1. Polypeptid mit Cellobiohydrolaseaktivität, wobei das Polypeptid eine Aminosäuresequenz mit mindestens 85% Sequenzidentität zu SEQ ID NO: 2 umfasst, wobei der Aminosäurerest in Position Q1 von SEQ ID NO: 2 durch Substitution oder Deletion modifiziert ist.

2. Polypeptid nach Anspruch 1, wobei das Polypeptid 50% seiner maximalen Stoffwandlungsleistung beibehält, wenn die Umwandlung 60 Minuten lang bei einer Temperatur von 60°C oder höher erfolgt.

3. Polypeptid nach Anspruch 1 oder 2, wobei das Polypeptid eine Aminosäuresequenz mit mindestens 90%, vorzugsweise mindestens 95%, stärker bevorzugt mindestens 99% Sequenzidentität zu SEQ ID NO: 2 umfasst.

4. Polypeptid nach einem oder mehreren der vorhergehenden Ansprüche, wobei die Aminosäuresequenz des Polypeptids die Sequenz gemäß SEQ ID NO: 2 oder eine Sequenz gemäß SEQ ID NO: 2, wobei 1 bis 75 Aminosäurereste, stärker bevorzugt 1 bis 35 Aminosäurereste, substituiert, deletiert oder insertiert sind, aufweist.

5. Polypeptid nach Anspruch 4, wobei zusätzlich einer oder mehrere der folgenden Aminosäurereste in der Sequenz gemäß SEQ ID NO: 2 durch Substitution oder Deletion modifiziert sind: die Positionen G4, A6, T15, Q28, W40, D64, E65, A72, S86, K92, V130, V152, Y155, K159, D181, E183, N194, D202, P224, T243, Y244, 1277, K304, N310, S311, N318, D320, T335, T344, D346, Q349, A358, Y374, A375, T392, T393, D410, Y422, P442, N445, R446, T456, S460, P462, G463, H468 und/oder V482 der Aminosäuren 1 bis 500 von SEQ ID NO: 2.

6. Polypeptid nach Anspruch 5, wobei das Polypeptid einen oder mehrere der folgenden bevorzugten Austausche in Bezug auf die Sequenz gemäß SEQ ID NO: 2 umfasst:
| Position | Bevorzugter Austausch |
|---|---|
| Q1 | L |
| G4 | C |
| A6 | G, V |
| T15 | S |
| Q28 | Q, R |
| W40 | R |
| D64 | N |
| E65 | K, V |
| A72 | C, V |
| S86 | T |
| K92 | K, R |
| V130 | I, V |
| V152 | A, E |
| Y155 | C |
| K159 | E |
| D181 | N |
| E183 | V, K |
| N194 | C, R, Y, D, K, I, L, G, Q, S, V |
| D202 | Y, N, G |
| P224 | L |
| T243 | I, C, R, Y, A, F, Q, P, D, V, W, L, M |
| Y244 | F, H |
| 1277 | V |
| K304 | R |
| N310 | D |
| S311 | G, N |
| N318 | Y |
| D320 | V, E, N |
| T335 | I |
| T344 | M |
| D346 | G, A, E, V |
| Q349 | R, K |
| A358 | E |
| Y374 | C, P, R, H, S, A |
| A375 | C, D, N, Y, R, Q, L, V, E, G, T, M |
| T392 | C, D, K |
| T393 | A |
| D410 | G |
| Y422 | F |
| P442 | S, del |
| N445 | D |
| R446 | S, G |
| T456 | T, A |
| S460 | L, P |
| P462 | L, del |
| G463 | D |
| H468 | L, Q, R |
| V482 | A, I |

7. Polypeptid nach einem oder mehreren der vorhergehenden Ansprüche, wobei das Polypeptid eine Aminosäuresequenz, ausgewählt aus der Liste der folgenden Mutationen von SEQ ID NO: 2, aufweist:
| | |
|---|---|
| 75 | Q1L, G4C, A72C, Q349K |
| 88 | Q1L, G4C, A72C, S86T, Q349R |
| 89 | Q1L, G4C, A72C, D181N, Q349R |
| 90 | Q1L, G4C, A72C, E183K, Q349R |
| 91 | Q1L, G4C, A72C, D181N, E183K, Q349R |
| 92 | Q1L, G4C, A72C, D320V, Q349R |
| 93 | Q1L, G4C, A72C, S86T, D181N, E183K, D320V, Q349R |
| 98 | Q1L, G4C, A72C, Q349R |
| 148 | Q1L, G4C, A72C, Q349K, T392M |
| 153 | Q1L, G4C, A68T, A72C, Q349K, G439D, R453S |
| 154 | Q1L, G4C, A72C, D202N, Q349K |
| 155 | Q1L, G4C, A68T, A72C, Q349K |
| 156 | Q1L, G4C, A72C, K154R, Q349K, T3a3I |
| 157 | Q1L, G4C, A72C, S193P, Q349K, V482I |
| 158 | Q1L, G4C, A72C, H203R, Q349K, P442S |
| 159 | Q1L, G4C, A72C, Q349K, H468R |
| 160 | Q1L, G4C, A72C, D202N, Q349K, G486D |
| 161 | Q1L, G4C, E65K, A72C, Q349K |
| 162 | Q1L, G4C, A72C, Q349K, Y422F |
| 163 | Q1L, G4C, Q28R, A72C, Q349K, H468L |
| 164 | Q1L, G4C, A72C, D181N, D247N, Q349K |
| 165 | Q1L, G4C, A72C, D181N, Q349K, T451S |
| 166 | Q1L, G4C, Q28R, A72C, Q349K |
| 167 | Q1L, G4C, A72C, A145T, H203R, Q349K, T403K |
| 168 | Q1L, G4C, A72C, I200F, Q349K, L500I |
| 169 | Q1L, G4C, D64N, A72C, Q349K |
| 170 | Q1L, G4C, A72C, V152A, Q349K |
| 171 | Q1L, G4C, T15S, A72C, Y244F, Q349K |
| 172 | Q1L, G4C, A6V, A72C, Q349K |
| 173 | Q1L, G4C, A72C, S311N, Q349K, G463D |
| 174 | Q1L, G4C, A72C, Y155C, Q349K |
| 175 | Q1L, G4C, A72C, S311N, Q349K |
| 176 | Q1L, G4C, A72C, D346V, Q349K |
| 177 | Q1L, G4C, A72C, Q349K, T392K |
| 178 | Q1L, G4C, A72C, S311G, Q349K |
| 179 | Q1L, G4C, A72C, S311G, Q349K, H468R |

8. Polypeptid nach einem oder mehreren der vorhergehenden Ansprüche, das nach dem Einführen einer Nukleinsäure, die für ein Polypeptid mit einer Aminosäuresequenz mit mindestens 85% Sequenzidentität zu der SEQ ID NO: 2 in eine Hefe, wobei der Aminosäurerest in Position Q1 von SEQ ID NO: 2 durch Substitution oder Deletion modifiziert sein muss, auf einem Niveau von mehr als 100 mg/l, stärker bevorzugt mehr als 200 mg/l, besonders bevorzugt mehr als 500 mg/l und am stärksten bevorzugt mehr als 1 g/l exprimiert und in den Überstand sezerniert wird.

9. Nukleinsäure, die für das Polypeptid nach einem oder mehreren der Ansprüche 1 bis 8 codiert, vorzugsweise mit mindestens 95% Identität zu SEQ ID NO: 1.

10. Vektor, der die Nukleinsäure nach Anspruch 9 umfasst.

11. Wirtszelle, die mit einem Vektor nach Anspruch 10 transformiert ist.

12. Wirtszelle nach Anspruch 11, wobei die Wirtszelle von der Gruppe bestehend aus Saccharomyces, Schizosaccharomyces, Kluyveromyces, Pichia, Hansenula, Aspergillus, Trichoderma, Penicillium, Candida und Yarrowina abgeleitet ist.

13. Zusammensetzung, umfassend das Polypeptid nach einem oder mehreren der Ansprüche 1 bis 9 und eine oder mehrere Endoglucanasen und/oder eine oder mehrere beta-Glucosidasen und/oder eine oder mehrere weitere Cellobiohydrolasen und/oder eine oder mehrere Xylanasen.

14. Verwendung des Polypeptids nach einem oder mehreren der Ansprüche 1 bis 9 oder der Zusammensetzung nach Anspruch 13 für den enzymatischen Abbau von Lignocellulosebiomasse und/oder für die Textilverarbeitung und/oder als Bestandteil in Detergenzien und/oder als Bestandteil in Nahrungs- oder Futtermittelzusammensetzungen.

## Revendications

1. Polypeptide ayant une activité de cello-biohydrolase, dans laquelle le polypeptide comprend une séquence d'acides aminés ayant une identité de séquence, d'au moins 85%, avec la SEQ ID n° : 2, dans lequel le résidu d'acide aminé en position Q1 de la SEQ ID n° : 2 est modifié par substitution ou délétion.

2. Polypeptide selon la revendication 1, dans laquelle le polypeptide conserve 50% de sa capacité maximale de conversion du substrat quand la conversion est faite pendant 60 minutes à une température de 60°C ou plus.

3. Polypeptide selon la revendication 1 ou la 2, dans laquelle le polypeptide comprend une séquence d'acides aminés ayant une identité de séquence d'au moins 90%, de préférence d'au moins 95%, mieux préféré d'au moins 99%, avec la SEQ ID n° : 2.

4. Polypeptide selon une ou plusieurs des revendications précédentes, dans laquelle la séquence d'acides aminés du polypeptide a la séquence définie par la SEQ ID n° : 2 ou une séquence telle que définie par la SEQ ID n° : 2, dans laquelle de 1 à 75 résidu(s) d'acide(s) aminé(s), mieux préféré de 1 à 35 résidu(s) d'acide(s) aminé(s) est (sont) substitué(s), supprimé(s) ou inséré(s).

5. Polypeptide selon la revendication 4, dans laquelle en outre un ou plusieurs des résidus d'acides aminés suivants de la séquence définie par la SEQ ID n° : 2 est (sont) modifié(s) par substitution ou délétion : positions G4, A6, T15, Q28, W40, D64, E65, A72, S86, K92, V130, V152, Y155, K159, D181, E183, N194, D202, P224, T243, Y244, 1277, K304, N310, S311, N318, D320, T335, T344, D346, Q349, A358, Y374, A375, T392, T393, D410, Y422, P442, N445, R446, T456, S460, P462, G463, H468 et/ou V482 des acides aminés 1 à 500 de la SEQ ID n° : 2.

6. Polypeptide selon la revendication 5, dans laquelle le polypeptide comprend un ou plusieurs des échanges préférés suivants en ce qui concerne la séquence définie par la SEQ ID n° : 2 :
| Position | Échange préféré |
|---|---|
| Q1 | L |
| G4 | C |
| A6 | G,V |
| T15 | S |
| Q28 | Q,R |
| W40 | R |
| D64 | N |
| E65 | K,V |
| A72 | C,V |
| S86 | T |
| K92 | K,R |
| V130 | I,V |
| V152 | A,E |
| Y155 | C |
| K159 | E |
| D181 | N |
| E183 | V,K |
| N194 | C,R,Y,D,K,I,L,G,Q,S,V |
| D202 | Y,N,G |
| P224 | L |
| T243 | I,C,R,Y,A,F,Q,P,D,V,W,L,M |
| Y244 | F,H |
| Í277 | V |
| K304 | R |
| N310 | D |
| S311 | G,N |
| N318 | Y |
| D320 | V,E,N |
| T335 | I |
| T344 | M |
| D346 | G,A,E,V |
| Q349 | R,K |
| A358 | E |
| Y374 | C,P,R,H,S,A |
| A375 | C,D,N,Y,R,Q,L,V,E,G,T,M |
| T392 | C,D,K |
| T393 | A |
| D410 | G |
| Y422 | F |
| P442 | S,del |
| N445 | D |
| R446 | S,G |
| T456 | T,A |
| S460 | L,P |
| P462 | L,del |
| G463 | D |
| H468 | L,Q,R |
| V482 | A,I |

7. Polypeptide selon une ou plusieurs des revendications précédentes, dans laquelle le polypeptide a une séquence d'acides aminés choisie dans la liste des mutations suivantes de la SEQ ID n° : 2 :
| | |
|---|---|
| 75 | Q1L,G4C,A72C,Q349K |
| 88 | Q1L,G4C,A72C,S86T,Q349R |
| 89 | Q1L,G4C,A72C,D181N,Q349R |
| 90 | Q1L,G4C,A72C,E183K,Q349R |
| 91 | Q1L,G4C,A72C,D181N,E183K,Q349R |
| 92 | Q1L,G4C,A72C,D320V,Q349R |
| 93 | Q1L,G4C,A72C,S86T,D181N,E183K,D320V,Q349R |
| 98 | Q1L,G4C,A72C,Q349R |
| 148 | Q1L,G4C,A72C,Q349K,T392M |
| 153 | Q1L,G4C,A68T,A72C,Q349K,G439D,R453S |
| 154 | Q1L,G4C,A72C,D202N,Q349K |
| 155 | Q1L,G4C,A68T,A72C,Q349K |
| 156 | Q1L,G4C,A72C,K154R,Q349K,T393I |
| 157 | Q1L,G4C,A72C,S193P,Q349K,V482I |
| 158 | Q1L,G4C,A72C,H203R,Q349K,P442S |
| 159 | Q1L,G4C,A72C,Q349K,H468R |
| 160 | Q1L,G4C,A72C,D202N,Q349K,G486D |
| 161 | Q1L,G4C,E65K,A72C,Q349K |
| 162 | Q1L,G4C,A72C,Q349K,Y422F |
| 163 | Q1L,G4C,Q28R,A72C,Q349K,H468L |
| 164 | Q1L,G4C,A72C,D181N,D247N,Q349K |
| 165 | Q1L,G4C,A72C,D181N,Q349K,T451S |
| 166 | Q1L,G4C,Q28R,A72C,Q349K |
| 167 | Q1L,G4C,A72C,A145T,H203R,Q349K,T403K |
| 168 | Q1L,G4C,A72C,I200F,Q349K,L500I |
| 169 | Q1L,G4C,D64N,A72C,Q349K |
| 170 | Q1L,G4C,A72C,V152A,Q349K |
| 171 | Q1L,G4C,T15S,A72C,Y244F,Q349K |
| 172 | Q1L,G4C,A6V,A72C,Q349K |
| 173 | Q1L,G4C,A72C,S311N,Q349K,G463D |
| 174 | Q1L,G4C,A72C,Y155C,Q349K |
| 175 | Q1L,G4C,A72C,S311N,Q349K |
| 176 | Q1L,G4C,A72C,D346V,Q349K |
| 177 | Q1L,G4C,A72C,Q349K,T392K |
| 178 | Q1L,G4C,A72C,S311G,Q349K |
| 179 | Q1L,G4C,A72C,S311G,Q349K,H468R |

8. Polypeptide, selon une ou plusieurs des revendications précédentes, qui est exprimé et sécrété à un taux supérieur à 100 mg/l, mieux préféré supérieur à 200 mg/l, particulièrement préféré supérieur à 500 mg/l et, de manière préférée entre toutes, supérieur à 1 g/l dans le surnageant après l'introduction d'un acide nucléique, qui code pour un polypeptide ayant une séquence d'acides aminés avec une identité de séquence d'au moins 85% avec la SEQ ID n° : 2, dans une levure, dans laquelle le résidu d'acide aminé en position Q1 de la SEQ ID n° : 2 doit être modifié par substitution ou délétion.

9. Acide nucléique codant pour le polypeptide, selon une ou plusieurs des revendications 1 à 8, ayant de préférence une identité d'au moins 95% avec la SEQ ID n° : 1.

10. Vecteur comprenant l'acide nucléique selon la revendication 9.

11. Cellule hôte transformée avec un vecteur selon la revendication 10.

12. Cellule hôte selon la revendication 11, dans laquelle la cellule hôte est dérivée du groupe constitué par Saccharomyces, Schizosaccharomyces, Kluyveromyces, Pichia, Hansenula, Aspergillus, Trichoderma, Penicillium, Candida et Yarrowina.

13. Composition comprenant le polypeptide, selon une ou plusieurs des revendications 1 à 9, et une ou plusieurs endoglucanase(s) et/ou une ou plusieurs bêta-glucosidase(s) et/ou une ou plusieurs cello-biohydrolase(s) en outre et/ou une ou plusieurs xylanase(s).

14. Utilisation du polypeptide, selon une ou plusieurs des revendications 1 à 9, ou de la composition, selon la revendication 13, pour la dégradation enzymatique d'une biomasse ligno-cellulosique, et/ou pour le traitement de textiles et/ou comme ingrédient dans les détergents et/ou comme ingrédient dans de la nourriture ou des compositions alimentaires.


### SEQUENCE LISTING

<110> Süd-Chemie AG
<120> optimized Cellulase Enzymes
<130> 139 202
<160> 41
<170> PatentIn version 3.4
<210> 1
   <211> 1509
   <212> DNA
   <213> Artificial
<220>
   <223> Coding Sequence for Talaromyces emersonii CBHI /Trichoderma reesei -CBD fusion (mature CBH-a)
<400> 1
   cagcaggccg gcacggcgac ggcagagaac cacccgcccc tgacatggca ggaatgcacc      60
   gcccctggga gctgcaccac ccagaacggg gcggtcgttc ttgatgcgaa ctggcgttgg     120
   gtgcacgatg tgaacggata caccaactgc tacacgggca atacctggga ccccacgtac     180
   tgccctgacg acgaaacctg cgcccagaac tgtgcgctgg acggcgcgga ttacgagggc     240
   acctacggcg tgacttcgtc gggcagctcc ttgaaactca atttcgtcac cgggtcgaac     300
   gtcggatccc gtctctacct gctgcaggac gactcgacct atcagatctt caagctcctg     360
   aaccgcgagt tcagctttga cgtcgatgtc tccaatcttc cgtgcggatt gaacggcgct     420
   ctgtactttg tcgccatgga cgccgacggc ggcgtgtcca agtacccgaa caacaaggct     480
   ggtgccaagt acggaaccgg gtattgcgac tcccaatgcc cacgggacct caagttcatc     540
   gacggcgagg ccaacgtcga gggctggcag ccgtcttcga acaacgccaa caccggaatt     600
   ggcgaccacg gctcctgctg tgcggagatg gatgtctggg aagcaaacag catctccaat     660
   gcggtcactc cgcacccgtg cgacacgcca ggccagacga tgtgctctgg agatgactgc     720
   ggtggcacat actctaacga tcgctacgcg ggaacctgcg atcctgacgg ctgtgacttc     780
   aacccttacc gcatgggcaa cacttctttc tacgggcctg gcaagatcat cgataccacc     840
   aagcccttca ctgtcgtgac gcagttcctc actgatgatg gtacggatac tggaactctc     900
   agcgagatca agcgcttcta catccagaac agcaacgtca ttccgcagcc caactcggac     960
   atcagtggcg tgaccggcaa ctcgatcacg acggagttct gcactgctca gaagcaggcc    1020
   tttggcgaca cggacgactt ctctcagcac ggtggcctgg ccaagatggg agcggccatg    1080
   cagcagggta tggtcctggt gatgagtttg tgggacgact acgccgcgca gatgctgtgg    1140
   ttggattccg actacccgac ggatgcggac cccacgaccc ctggtattgc ccgtggaacg    1200
   tgtccgacgg actcgggcgt cccatcggat gtcgagtcgc agagccccaa ctcctacgtg    1260
   acctactcga acattaagtt tggtccgatc ggtagcacag gtaatccttc aggtggtaat    1320
   cctccaggtg gaaacagagg aacaacgaca actagaagac cagctactac aactggttca    1380
   agtccaggtc caactcaatc acactacggt caatgtggtg gtataggtta ctctggtccc    1440
   actgtttgtg cttctggtac tacttgccaa gttctgaacc cttactactc acagtgtcta    1500
   taatgataa                                                            1509
<210> 2
   <211> 500
   <212> PRT
   <213> Artificial
<220>
   <223> Mature sequence of Talaromyces emersonii CBHI / Trichoderma reesei -CBD (mature CBH-a)
<400> 2
   Gln Gln Ala Gly Thr Ala Thr Ala Glu Asn His Pro Pro Leu Thr Trp
   1               5                   10                  15
   Gln Glu Cys Thr Ala Pro Gly Ser Cys Thr Thr Gln Asn Gly Ala Val
               20                  25                  30
   Val Leu Asp Ala Asn Trp Arg Trp Val His Asp Val Asn Gly Tyr Thr
           35                  40                  45
   Asn Cys Tyr Thr Gly Asn Thr Trp Asp Pro Thr Tyr Cys Pro Asp Asp
       50                  55                  60
   Glu Thr Cys Ala Gln Asn Cys Ala Leu Asp Gly Ala Asp Tyr Glu Gly
   65                  70                  75                  80
   Thr Tyr Gly Val Thr Ser Ser Gly Ser Ser Leu Lys Leu Asn Phe Val
                   85                  90                  95
   Thr Gly Ser Asn Val Gly Ser Arg Leu Tyr Leu Leu Gln Asp Asp Ser
               100                 105                 110
   Thr Tyr Gln Ile Phe Lys Leu Leu Asn Arg Glu Phe Ser Phe Asp Val
           115                 120                 125
   Asp Val Ser Asn Leu Pro Cys Gly Leu Asn Gly Ala Leu Tyr Phe Val
       130                 135                 140
   Ala Met Asp Ala Asp Gly Gly Val Ser Lys Tyr Pro Asn Asn Lys Ala
   145                 150                 155                 160
   Gly Ala Lys Tyr Gly Thr Gly Tyr Cys Asp Ser Gln Cys Pro Arg Asp
                   165                 170                 175
   Leu Lys Phe Ile Asp Gly Glu Ala Asn Val Glu Gly Trp Gln Pro Ser
               180                 185                 190
   Ser Asn Asn Ala Asn Thr Gly Ile Gly Asp His Gly Ser Cys Cys Ala
           195                 200                 205
   Glu Met Asp Val Trp Glu Ala Asn Ser Ile Ser Asn Ala Val Thr Pro
   210 215 220
   His Pro Cys Asp Thr Pro Gly Gln Thr Met Cys Ser Gly Asp Asp Cys
   225 230 235 240
   Gly Gly Thr Tyr Ser Asn Asp Arg Tyr Ala Gly Thr Cys Asp Pro Asp
   245 250 255
   Gly Cys Asp Phe Asn Pro Tyr Arg Met Gly Asn Thr Ser Phe Tyr Gly
   260 265 270
   Pro Gly Lys Ile Ile Asp Thr Thr Lys Pro Phe Thr Val Val Thr Gln
   275 280 285
   Phe Leu Thr Asp Asp Gly Thr Asp Thr Gly Thr Leu Ser Glu Ile Lys
   290 295 300
   Arg Phe Tyr Ile Gln Asn Ser Asn Val Ile Pro Gln Pro Asn Ser Asp
   305 310 315 320
   Ile Ser Gly Val Thr Gly Asn Ser Ile Thr Thr Glu Phe Cys Thr Ala
   325 330 335
   Gln Lys Gln Ala Phe Gly Asp Thr Asp Asp Phe Ser Gln His Gly Gly
   340 345 350
   Leu Ala Lys Met Gly Ala Ala Met Gln Gln Gly Met Val Leu Val Met
   355 360 365
   Ser Leu Trp Asp Asp Tyr Ala Ala Gln Met Leu Trp Leu Asp Ser Asp
   370 375 380
   Tyr Pro Thr Asp Ala Asp Pro Thr Thr Pro Gly Ile Ala Arg Gly Thr
   385 390 395 400
   Cys Pro Thr Asp Ser Gly Val Pro Ser Asp Val Glu Ser Gln Ser Pro
   405 410 415
   Asn Ser Tyr Val Thr Tyr Ser Asn Ile Lys Phe Gly Pro Ile Gly Ser
   420 425 430
   Thr Gly Asn Pro Ser Gly Gly Asn Pro Pro Gly Gly Asn Arg Gly Thr
   435 440 445
   Thr Thr Thr Arg Arg Pro Ala Thr Thr Thr Gly Ser Ser Pro Gly Pro
   450 455 460
   Thr Gln Ser His Tyr Gly Gln Cys Gly Gly Ile Gly Tyr Ser Gly Pro
   465 470 475 480
   Thr Val Cys Ala Ser Gly Thr Thr Cys Gln Val Leu Asn Pro Tyr Tyr
   485 490 495
   Ser Gln Cys Leu
   500
<210> 3
   <211> 1581
   <212> DNA
   <213> Artificial
<220>
   <223> Coding sequence of the fusion of CBH-a with Trichoderma reesei CBH Signal peptide
<400> 3
   atgtatcgga agttggccgt catctcggcc ttcttggcca cagctcgtgc tcagcaggcc      60
   ggcacggcga cggcagagaa ccacccgccc ctgacatggc aggaatgcac cgcccctggg     120
   agctgcacca cccagaacgg ggcggtcgtt cttgatgcga actggcgttg ggtgcacgat     180
   gtgaacggat acaccaactg ctacacgggc aatacctggg accccacgta ctgccctgac     240
   gacgaaacct gcgcccagaa ctgtgcgctg gacggcgcgg attacgaggg cacctacggc     300
   gtgacttcgt cgggcagctc cttgaaactc aatttcgtca ccgggtcgaa cgtcggatcc     360
   cgtctctacc tgctgcagga cgactcgacc tatcagatct tcaagctcct gaaccgcgag     420
   ttcagctttg acgtcgatgt ctccaatctt ccgtgcggat tgaacggcgc tctgtacttt     480
   gtcgccatgg acgccgacgg cggcgtgtcc aagtacccga acaacaaggc tggtgccaag     540
   tacggaaccg ggtattgcga ctcccaatgc ccacgggacc tcaagttcat cgacggcgag     600
   gccaacgtcg agggctggca gccgtcttcg aacaacgcca acaccggaat tggcgaccac     660
   ggctcctgct gtgcggagat ggatgtctgg gaagcaaaca gcatctccaa tgcggtcact     720
   ccgcacccgt gcgacacgcc aggccagacg atgtgctctg gagatgactg cggtggcaca     780
   tactctaacg atcgctacgc gggaacctgc gatcctgacg gctgtgactt caacccttac     840
   cgcatgggca acacttcttt ctacgggcct ggcaagatca tcgataccac caagcccttc     900
   actgtcgtga cgcagttcct cactgatgat ggtacggata ctggaactct cagcgagatc     960
   aagcgcttct acatccagaa cagcaacgtc attccgcagc ccaactcgga catcagtggc    1020
   gtgaccggca actcgatcac gacggagttc tgcactgctc agaagcaggc ctttggcgac    1080
   acggacgact tctctcagca cggtggcctg gccaagatgg gagcggccat gcagcagggt    1140
   atggtcctgg tgatgagttt gtgggacgac tacgccgcgc agatgctgtg gttggattcc    1200
   gactacccga cggatgcgga ccccacgacc cctggtattg cccgtggaac gtgtccgacg    1260
   gactcgggcg tcccatcgga tgtcgagtcg cagagcccca actcctacgt gacctactcg    1320
   aacattaagt ttggtccgat cggtagcaca ggtaatcctt caggtggtaa tcctccaggt    1380
   ggaaacagag gaacaacgac aactagaaga ccagctacta caactggttc aagtccaggt    1440
   ccaactcaat cacactacgg tcaatgtggt ggtataggtt actctggtcc cactgtttgt    1500
   gcttctggta ctacttgcca agttctgaac ccttactact cacagtgtct agcttctgca    1560
   catcatcacc accaccatta a                                               1581
<210> 4
   <211> 70
   <212> PRT
   <213> Artificial
<220>
   <223> Trichoderma reesei CBHI cellulose binding domain and linker sequence
<400> 4
   Gly Ser Thr Gly Asn Pro Ser Gly Gly Asn Pro Pro Gly Gly Asn Arg
   1 5 10 15
   Gly Thr Thr Thr Thr Arg Arg Pro Ala Thr Thr Thr Gly Ser Ser Pro
   20 25 30
   Gly Pro Thr Gln Ser His Tyr Gly Gln Cys Gly Gly Ile Gly Tyr Ser
   35 40 45
   Gly Pro Thr Val Cys Ala Ser Gly Thr Thr Cys Gln Val Leu Asn Pro
   50 55 60
   Tyr Tyr Ser Gln Cys Leu
   65 70
<210> 5
   <211> 437
   <212> PRT
   <213> Artificial
<220>
   <223> Talaromyces emersonii CBHI sequence (CBH-b)
<400> 5
   Gln Gln Ala Gly Thr Ala Thr Ala Glu Asn His Pro Pro Leu Thr Trp
   1 5 10 15
   Gln Glu Cys Thr Ala Pro Gly Ser Cys Thr Thr Gln Asn Gly Ala Val
   20 25 30
   Val Leu Asp Ala Asn Trp Arg Trp Val His Asp Val Asn Gly Tyr Thr
   35 40 45
   Asn Cys Tyr Thr Gly Asn Thr Trp Asp Pro Thr Tyr Cys Pro Asp Asp
   50 55 60
   Glu Thr Cys Ala Gln Asn Cys Ala Leu Asp Gly Ala Asp Tyr Glu Gly
   65 70 75 80
   Thr Tyr Gly Val Thr Ser Ser Gly Ser Ser Leu Lys Leu Asn Phe Val
   85 90 95
   Thr Gly Ser Asn Val Gly Ser Arg Leu Tyr Leu Leu Gln Asp Asp Ser
   100 105 110
   Thr Tyr Gln Ile Phe Lys Leu Leu Asn Arg Glu Phe Ser Phe Asp Val
   115 120 125
   Asp Val Ser Asn Leu Pro Cys Gly Leu Asn Gly Ala Leu Tyr Phe Val
   130 135 140
   Ala Met Asp Ala Asp Gly Gly Val Ser Lys Tyr Pro Asn Asn Lys Ala
   145 150 155 160
   Gly Ala Lys Tyr Gly Thr Gly Tyr Cys Asp Ser Gln Cys Pro Arg Asp
   165 170 175
   Leu Lys Phe Ile Asp Gly Glu Ala Asn Val Glu Gly Trp Gln Pro Ser
   180 185 190
   Ser Asn Asn Ala Asn Thr Gly Ile Gly Asp His Gly Ser Cys Cys Ala
   195 200 205
   Glu Met Asp Val Trp Glu Ala Asn Ser Ile Ser Asn Ala Val Thr Pro
   210 215 220
   His Pro Cys Asp Thr Pro Gly Gln Thr Met Cys Ser Gly Asp Asp Cys
   225 230 235 240
   Gly Gly Thr Tyr Ser Asn Asp Arg Tyr Ala Gly Thr Cys Asp Pro Asp
   245 250 255
   Gly Cys Asp Phe Asn Pro Tyr Arg Met Gly Asn Thr Ser Phe Tyr Gly
   260 265 270
   Pro Gly Lys Ile Ile Asp Thr Thr Lys Pro Phe Thr Val Val Thr Gln
   275 280 285
   Phe Leu Thr Asp Asp Gly Thr Asp Thr Gly Thr Leu Ser Glu Ile Lys
   290 295 300
   Arg Phe Tyr Ile Gln Asn Ser Asn Val Ile Pro Gln Pro Asn Ser Asp
   305 310 315 320
   Ile Ser Gly Val Thr Gly Asn Ser Ile Thr Thr Glu Phe Cys Thr Ala
   325 330 335
   Gln Lys Gln Ala Phe Gly Asp Thr Asp Asp Phe Ser Gln His Gly Gly
   340 345 350
   Leu Ala Lys Met Gly Ala Ala Met Gln Gln Gly Met Val Leu Val Met
   355 360 365
   Ser Leu Trp Asp Asp Tyr Ala Ala Gln Met Leu Trp Leu Asp Ser Asp
   370 375 380
   Tyr Pro Thr Asp Ala Asp Pro Thr Thr Pro Gly Ile Ala Arg Gly Thr
   385                 390                 395                 400
   Cys Pro Thr Asp Ser Gly Val Pro Ser Asp Val Glu Ser Gln Ser Pro
                   405                 410                 415
   Asn Ser Tyr Val Thr Tyr Ser Asn Ile Lys Phe Gly Pro Ile Asn Ser
               420                 425                 430
   Thr Phe Thr Ala Ser
           435
<210> 6
   <211> 1590
   <212> DNA
   <213> Artificial
<220>
   <223> Coding sequence of Talaromyces emersonii CBHI fused to the alpha factor signal peptide
<400> 6
   atgagatttc cttcaatttt tactgcagtt ttattcgcag catcctccgc attagctgct      60
   ccagtcaaca ctacaacaga agatgaaacg gcacaaattc cggctgaagc tgtcatcggt     120
   tacttagatt tagaagggga tttcgatgtt gctgttttgc cattttccaa cagcacaaat     180
   aacgggttat tgtttataaa tactactatt gccagcattg ctgctaaaga agaaggggta     240
   tctttggata aacgtgaggc ggaagcaccc tctcagcagg ccggcacggc gacggcagag     300
   aaccacccgc ccctgacatg gcaggaatgc accgcccctg ggagctgcac cacccagaac     360
   ggggcggtcg ttcttgatgc gaactggcgt tgggtgcacg atgtgaacgg atacaccaac     420
   tgctacacgg gcaatacctg ggaccccacg tactgccctg acgacgaaac ctgcgcccag     480
   aactgtgcgc tggacggcgc ggattacgag ggcacctacg gcgtgacttc gtcgggcagc     540
   tccttgaaac tcaatttcgt caccgggtcg aacgtcggat cccgtctcta cctgctgcag     600
   gacgactcga cctatcagat cttcaagctt ctgaaccgcg agttcagctt tgacgtcgat     660
   gtctccaatc ttccgtgcgg attgaacggc gctctgtact ttgtcgccat ggacgccgac     720
   ggcggcgtgt ccaagtaccc gaacaacaag gctggtgcca agtacggaac cgggtattgc     780
   gactcccaat gcccacggga cctcaagttc atcgacggcg aggccaacgt cgagggctgg     840
   cagccgtctt cgaacaacgc caacaccgga attggcgacc acggctcctg ctgtgcggag     900
   atggatgtct gggaagcaaa cagcatctcc aatgcggtca ctccgcaccc gtgcgacacg     960
   ccaggccaga cgatgtgctc tggagatgac tgcggtggca catactctaa cgatcgctac    1020
   gcgggaacct gcgatcctga cggctgtgac ttcaaccctt accgcatggg caacacttct    1080
   ttctacgggc ctggcaagat catcgatacc accaagccct tcactgtcgt gacgcagttc    1140
   ctcactgatg atggtacgga tactggaact ctcagcgaga tcaagcgctt ctacatccag    1200
   aacagcaacg tcattccgca gcccaactcg gacatcagtg gcgtgaccgg caactcgatc    1260
   acgacggagt tctgcactgc tcagaagcag gcctttggcg acacggacga cttctctcag    1320
   cacggtggcc tggccaagat gggagcggcc atgcagcagg gtatggtcct ggtgatgagt    1380
   ttgtgggacg actacgccgc gcagatgctg tggttggatt ccgactaccc gacggatgcg    1440
   gaccccacga cccctggtat tgcccgtgga acgtgtccga cggactcggg cgtcccatcg    1500
   gatgtcgagt cgcagagccc caactcctac gtgacctact cgaacattaa gtttggtccg    1560
   atcaactcga ccttcaccgc ttcgtgataa                                     1590
<210> 7
   <211> 429
   <212> PRT
   <213> Artificial
<220>
   <223> Humicola grisea CBHI (CBH-d)
<400> 7
   Gln Gln Ala Gly Thr Ile Thr Ala Glu Asn His Pro Arg Met Thr Trp
   1 5 10 15
   Lys Arg Cys Ser Gly Pro Gly Asn Cys Gln Thr Val Gln Gly Glu Val
   20 25 30
   Val Ile Asp Ala Asn Trp Arg Trp Leu His Asn Asn Gly Gln Asn Cys
   35 40 45
   Tyr Glu Gly Asn Lys Trp Thr Ser Gln Cys Ser Ser Ala Thr Asp Cys
   50 55 60
   Ala Gln Arg Cys Ala Leu Asp Gly Ala Asn Tyr Gln Ser Thr Tyr Gly
   65 70 75 80
   Ala Ser Thr Ser Gly Asp Ser Leu Thr Leu Lys Phe Val Thr Lys His
   85 90 95
   Glu Tyr Gly Thr Asn Ile Gly Ser Arg Phe Tyr Leu Met Ala Asn Gln
   100 105 110
   Asn Lys Tyr Gln Met Phe Thr Leu Met Asn Asn Glu Phe Ala Phe Asp
   115 120 125
   Val Asp Leu Ser Lys Val Glu Cys Gly Ile Asn Ser Ala Leu Tyr Phe
   130 135 140
   Val Ala Met Glu Glu Asp Gly Gly Met Ala Ser Tyr Pro Ser Asn Arg
   145 150 155 160
   Ala Gly Ala Lys Tyr Gly Thr Gly Tyr Cys Asp Ala Gln Cys Ala Arg
   165 170 175
   Asp Leu Lys Phe Ile Gly Gly Lys Ala Asn Ile Glu Gly Trp Arg Pro
   180 185 190
   Ser Thr Asn Asp Pro Asn Ala Gly Val Gly Pro Met Gly Ala Cys Cys
   195 200 205
   Ala Glu Ile Asp Val Trp Glu Ser Asn Ala Tyr Ala Tyr Ala Phe Thr
   210 215 220
   Pro His Ala Cys Gly Ser Lys Asn Arg Tyr His Ile Cys Glu Thr Asn
   225 230 235 240
   Asn Cys Gly Gly Thr Tyr Ser Asp Asp Arg Phe Ala Gly Tyr Cys Asp
   245 250 255
   Ala Asn Gly Cys Asp Tyr Asn Pro Tyr Arg Met Gly Asn Lys Asp Phe
   260 265 270
   Tyr Gly Lys Gly Lys Thr Val Asp Thr Asn Arg Lys Phe Thr Val Val
   275 280 285
   Ser Arg Phe Glu Arg Asn Arg Leu Ser Gln Phe Phe Val Gln Asp Gly
   290 295 300
   Arg Lys Ile Glu Val Pro Pro Pro Thr Trp Pro Gly Leu Pro Asn Ser
   305 310 315 320
   Ala Asp Ile Thr Pro Glu Leu Cys Asp Ala Gln Phe Arg Val Phe Asp
   325 330 335
   Asp Arg Asn Arg Phe Ala Glu Thr Gly Gly Phe Asp Ala Leu Asn Glu
   340 345 350
   Ala Leu Thr Ile Pro Met Val Leu Val Met Ser Ile Trp Asp Asp His
   355 360 365
   His Ser Asn Met Leu Trp Leu Asp Ser Ser Tyr Pro Pro Glu Lys Ala
   370 375 380
   Gly Leu Pro Gly Gly Asp Arg Gly Pro Cys Pro Thr Thr Ser Gly Val
   385 390 395 400
   Pro Ala Glu Val Glu Ala Gln Tyr Pro Asp Ala Gln Val Val Trp Ser
   405 410 415
   Asn Ile Arg Phe Gly Pro Ile Gly Ser Thr Val Asn Val
   420 425
<210> 8
   <211> 1563
   <212> DNA
   <213> Artificial
<220>
   <223> Coding sequence of **Humicola** grisea CBHI fused to the alpha factor signal peptide
<400> 8
   atgagatttc cttcaatttt tactgcagtt ttattcgcag catcctccgc attagctgct     60
   ccagtcaaca ctacaacaga agatgaaacg gcacaaattc cggctgaagc tgtcatcggt    120
   tacttagatt tagaagggga tttcgatgtt gctgttttgc cattttccaa cagcacaaat    180
   aacgggttat tgtttataaa tactactatt gccagcattg ctgctaaaga agaaggggta    240
   tctttggata aacgtgaggc ggaagcaccc tctcagcagg ctggtactat tactgctgag    300
   aaccacccaa gaatgacctg gaagagatgc tctggtccag gaaactgtca gactgttcag    360
   ggcgaggttg tgattgacgc taattggaga tggttgcaca acaacggcca gaactgttac    420
   gagggtaaca agtggacctc tcagtgttct tctgctaccg actgtgctca gagatgtgct    480
   ttggacggtg ccaactacca gtctacctac ggtgcttcta cctctggtga ctctctgacc    540
   ctgaagttcg ttaccaagca cgagtacgga accaacatcg gctctagatt ctacctgatg    600
   gccaaccaga acaagtacca gatgttcacc ctgatgaaca acgagttcgc ctttgacgtt    660
   gacctgtcta aggtggagtg cggtatcaac tctgccctgt acttcgttgc tatggaagag    720
   gacggtggaa tggcttctta cccatctaac agagccggtg ctaagtacgg tactggttac    780
   tgtgacgccc agtgtgctag agacctgaag ttcatcggtg gaaaggccaa cattgagggt    840
   tggagaccat ctaccaacga cccaaacgct ggtgttggtc caatgggagc ttgttgtgcc    900
   gagattgatg tgtgggagtc taacgcttac gcctacgctt ttaccccaca cgcttgcggt    960
   tctaagaaca gataccacat ctgcgagacc aacaactgtg gtggaaccta ctctgacgac   1020
   agattcgctg gatactgcga cgctaacggt tgtgactaca acccatacag aatgggcaac   1080
   aaggacttct acggcaaggg aaagaccgtt gacaccaaca gaaagttcac cgtggtgtcg   1140
   agattcgaga gaaacagact gtcgcagttc tttgtgcagg acggcagaaa gattgaggtc   1200
   ccaccaccaa cttggccagg attgccaaac tctgccgaca ttaccccaga gttgtgtgac   1260
   gctcagttca gagtgttcga cgacagaaac agatttgctg agaccggtgg ttttgacgct   1320
   ttgaacgagg ctctgaccat tccaatggtg ctggtgatgt ctatttggga cgaccaccac   1380
   tctaacatgt tgtggctgga ctcttcttac ccaccagaga aggctggatt gccaggtggt   1440
   gacagaggac catgtccaac tacttcgggt gttccagctg aggttgaggc tcagtaccca   1500
   gacgctcagg ttgtgtggtc gaacatcaga ttcggcccaa tcggttctac cgtgaacgtg   1560
   taa                                                                  1563
<210> 9
   <211> 439
   <212> PRT
   <213> Artificial
<220>
   <223> Thermoascus auratiacus CBHI (CBH-e)
<400> 9
   His Glu Ala Gly Thr Val Thr Ala Glu Asn His Pro Ser Leu Thr Trp
   1 5 10 15
   Gln Gln Cys Ser Ser Gly Gly Ser Cys Thr Thr Gln Asn Gly Lys Val
   20 25 30
   Val Ile Asp Ala Asn Trp Arg Trp Val His Thr Thr Ser Gly Tyr Thr
   35 40 45
   Asn Cys Tyr Thr Gly Asn Thr Trp Asp Thr Ser Ile Cys Pro Asp Asp
   50 55 60
   Val Thr Cys Ala Gln Asn Cys Ala Leu Asp Gly Ala Asp Tyr Ser Gly
   65 70 75 80
   Thr Tyr Gly Val Thr Thr Ser Gly Asn Ala Leu Arg Leu Asn Phe Val
   85 90 95
   Thr Gln Ser Ser Gly Lys Asn Ile Gly Ser Arg Leu Tyr Leu Leu Gln
   100 105 110
   Asp Asp Thr Thr Tyr Gln Ile Phe Lys Leu Leu Gly Gln Glu Phe Thr
   115 120 125
   Phe Asp Val Asp Val Ser Asn Leu Pro Cys Gly Leu Asn Gly Ala Leu
   130 135 140
   Tyr Phe Val Ala Met Asp Ala Asp Gly Asn Leu Ser Lys Tyr Pro Gly
   145 150 155 160
   Asn Lys Ala Gly Ala Lys Tyr Gly Thr Gly Tyr Cys Asp Ser Gln Cys
   165 170 175
   Pro Arg Asp Leu Lys Phe Ile Asn Gly Gln Ala Asn Val Glu Gly Trp
   180 185 190
   Gln Pro Ser Ala Asn Asp Pro Asn Ala Gly Val Gly Asn His Gly Ser
   195 200 205
   Ser Cys Ala Glu Met Asp Val Trp Glu Ala Asn Ser Ile Ser Thr Ala
   210 215 220
   Val Thr Pro His Pro Cys Asp Thr Pro Gly Gln Thr Met Cys Gln Gly
   225 230 235 240
   Asp Asp Cys Gly Gly Thr Tyr Ser Ser Thr Arg Tyr Ala Gly Thr Cys
   245 250 255
   Asp Thr Asp Gly Cys Asp Phe Asn Pro Tyr Gln Pro Gly Asn His Ser
   260 265 270
   Phe Tyr Gly Pro Gly Lys Ile Val Asp Thr Ser Ser Lys Phe Thr Val
               275                 280                 285
   Val Thr Gln Phe Ile Thr Asp Asp Gly Thr Pro Ser Gly Thr Leu Thr
       290                 295                 300
   Glu Ile Lys Arg Phe Tyr Val Gln Asn Gly Lys Val Ile Pro Gln Ser
   305                 310                 315                 320
   Glu Ser Thr Ile Ser Gly Val Thr Gly Asn Ser Ile Thr Thr Glu Tyr
                   325                 330                 335
   Cys Thr Ala Gln Lys Ala Ala Phe Asp Asn Thr Gly Phe Phe Thr His
               340                 345                 350
   Gly Gly Leu Gln Lys Ile Ser Gln Ala Leu Ala Gln Gly Met Val Leu
           355                 360                 365
   Val Met Ser Leu Trp Asp Asp His Ala Ala Asn Met Leu Trp Leu Asp
       370                 375                 380
   Ser Thr Tyr Pro Thr Asp Ala Asp Pro Asp Thr Pro Gly Val Ala Arg
   385                 390                 395                 400
   Gly Thr Cys Pro Thr Thr Ser Gly Val Pro Ala Asp Val Glu Ser Gln
                   405                 410                 415
   Asn Pro Asn Ser Tyr Val Ile Tyr Ser Asn Ile Lys Val Gly Pro Ile
               420                 425                 430
   Asn Ser Thr Phe Thr Ala Asn
           435
<210> 10
   <211> 1593
   <212> DNA
   <213> Artificial
<220>
   <223> coding sequence of Thermoascus auratiacus CBHI fused to the alpha factor signal peptide
<400> 10
   atgagatttc cttcaatttt tactgcagtt ttattcgcag catcctccgc attagctgct      60
   ccagtcaaca ctacaacaga agatgaaacg gcacaaattc cggctgaagc tgtcatcggt     120
   tacttagatt tagaagggga tttcgatgtt gctgttttgc cattttccaa cagcacaaat     180
   aacgggttat tgtttataaa tactactatt gccagcattg ctgctaaaga agaaggggta     240
   tctttggata aacgtgaggc ggaagcaccc tctcacgagg ccggtaccgt aaccgcagag     300
   aatcaccctt ccctgacctg gcagcaatgc tccagcggcg gtagttgtac cacgcagaat     360
   ggaaaagtcg ttatcgatgc gaactggcgt tgggtccata ccacctctgg atacaccaac     420
   tgctacacgg gcaatacgtg ggacaccagt atctgtcccg acgacgtgac ctgcgctcag     480
   aattgtgcct tggatggagc ggattacagt ggcacctatg gtgttacgac cagtggcaac     540
   gccctgagac tgaactttgt cacccaaagc tcagggaaga acattggctc gcgcctgtac     600
   ctgctgcagg acgacaccac ttatcagatc ttcaagctgc tgggtcagga gtttaccttc     660
   gatgtcgacg tctccaatct cccttgcggg ctgaacggcg ccctctactt tgtggccatg     720
   gacgccgacg gcaatttgtc caaataccct ggcaacaagg caggcgctaa gtatggcact     780
   ggttactgcg actctcagtg ccctcgggat ctcaagttca tcaacggtca ggccaacgtt     840
   gaaggctggc agccgtctgc caacgaccca aatgccggcg ttggtaacca cggttcctcg     900
   tgcgctgaga tggatgtctg ggaagccaac agcatctcta ctgcggtgac gcctcaccca     960
   tgcgacaccc ccggccagac catgtgccag ggagacgact gtggtggaac ctactcctcc    1020
   actcgatatg ctggtacctg cgacactgat ggctgcgact tcaatcctta ccagccaggc    1080
   aaccactcgt tctacggccc cgggaagatc gtcgacacta gctccaaatt caccgtcgtc    1140
   acccagttca tcaccgacga cgggacaccc tccggcaccc tgacggagat caaacgcttc    1200
   tacgtccaga acggcaaggt gatcccccag tcggagtcga cgatcagcgg cgtcaccggc    1260
   aactcaatca ccaccgagta ttgcacggcc cagaaggcag ccttcgacaa caccggcttc    1320
   ttcacgcacg gcgggcttca gaagatcagt caggctctgg ctcagggcat ggtcctcgtc    1380
   atgagcctgt gggacgatca cgccgccaac atgctctggc tggacagcac ctacccgact    1440
   gatgcggacc cggacacccc tggcgtcgcg cgcggtacct gccccacgac ctccggcgtc    1500
   ccggccgacg tggagtcgca gaaccccaat tcatatgtta tctactccaa catcaaggtc    1560
   ggacccatca actcgacctt caccgccaac taa                                 1593
<210> 11
   <211> 1794
   <212> DNA
   <213> Artificial
<220>
   <223> Coding sequence for Trichoderma reesei CBHI (CBH-c), including the alpha factor signal peptide and a 6x His Tag
<400> 11
   atgagatttc cttcaatttt tactgcagtt ttattcgcag catcctccgc attagctgct      60
   ccagtcaaca ctacaacaga agatgaaacg gcacaaattc cggctgaagc tgtcatcggt     120
   tacttagatt tagaagggga tttcgatgtt gctgttttgc cattttccaa cagcacaaat     180
   aacgggttat tgtttataaa tactactatt gccagcattg ctgctaaaga agaaggggta     240
   tctttggata aacgtgaggc ggaagcaccc tcttcagctt gtacactgca atccgagact     300
   catccacctt taacgtggca aaagtgtagt tctggcggaa cttgtactca acagactggt     360
   agtgtcgtga tagatgctaa ctggagatgg acacatgcaa cgaactcctc aactaactgc     420
   tacgatggta acacctggtc ttctacattg tgtcctgaca acgaaacctg cgctaagaac     480
   tgttgtcttg atggagcagc ttacgcaagt acatatggtg tgactacctc tggtaacagc     540
   ctttccattg gttttgtaac ccagtcggct cagaagaatg ttggtgctag attgtacctg     600
   atggcttcag acaccacata ccaggagttt accttgttgg gaaacgagtt ctctttcgac     660
   gtagatgtgt ctcagctacc atgtggattg aatggagcct tgtactttgt ctcaatggat     720
   gcagacggag gtgtttcaaa gtaccctact aacacagctg gtgctaagta tggaactgga     780
   tactgcgatt ctcaatgccc aagagacctg aagttcatca acggacaagc taacgttgaa     840
   ggttgggaac cttctagcaa caacgcaaac actggaattg gtggtcatgg ttcttgctgt     900
   tcagagatgg acatttggga agccaactcc atcagtgaag ctttgactcc acatccatgc     960
   acaactgttg ggcaagaaat ttgcgaaggt gatggttgtg gtggcactta ctctgataac    1020
   agatacggcg gaacatgtga tccagatgga tgtgattgga acccatacag actgggtaac    1080
   acttcgtttt acggaccagg ttcttccttc actctagaca ctacgaagaa gttgactgtg    1140
   gtcacccaat ttgagacttc tggtgccatt aaccgatact acgtgcagaa cggagttact    1200
   ttccaacagc caaacgctga attgggtagt tactcaggca acgagcttaa cgatgactac    1260
   tgcactgctg aagaagcaga atttggtgga tcttcctttt cggataaggg tggattgacg    1320
   cagttcaaga aagctacctc tggtggaatg gttctagtca tgagtctgtg ggacgattac    1380
   tacgctaaca tgctttggct ggactctact taccctacaa acgagacatc ttctactcct    1440
   ggtgctgtaa gaggtagctg tttctacatc tctggagttc cagcccaagt tgagagtcaa   1500
   agtccaaatg ccaaggtcac cttctccaac atcaagttcg gaccaattgg tagcacaggt    1560
   aatccttcag gtggtaatcc tccaggtgga aacagaggaa caacgacaac tagaagacca    1620
   gctactacaa ctggttcaag tccaggtcca actcaatcac actacggtca atgtggtggt    1680
   ataggttact ctggtcccac tgtttgtgct tctggtacta cttgccaagt tctgaaccct   1740
   tactactcac agtgtctagc ttctgcacac catcatcatc atcattaatg ataa          1794
<210> 12
   <211> 496
   <212> PRT
   <213> Artificial
<220>
   <223> Trichoderma reesei CBHI (CBH-C)
<400> 12
   Gln Ser Ala Cys Thr Leu Gln Ser Glu Thr His Pro Pro Leu Thr Trp
   1 5 10 15
   Gln Lys Cys Ser Ser Gly Gly Thr Cys Thr Gln Gln Thr Gly Ser Val
   20 25 30
   Val Ile Asp Ala Asn Trp Arg Trp Thr His Ala Thr Asn Ser Ser Thr
   35 40 45
   Asn Cys Tyr Asp Gly Asn Thr Trp Ser Ser Thr Leu Cys Pro Asp Asn
   50 55 60
   Glu Thr Cys Ala Lys Asn Cys Cys Leu Asp Gly Ala Ala Tyr Ala Ser
   65 70 75 80
   Thr Tyr Gly Val Thr Thr Ser Gly Asn Ser Leu Ser Ile Gly Phe Val
   85 90 95
   Thr Gln Ser Ala Gln Lys Asn Val Gly Ala Arg Leu Tyr Leu Met Ala
   100 105 110
   Ser Asp Thr Thr Tyr Gln Glu Phe Thr Leu Leu Gly Asn Glu Phe Ser
   115 120 125
   Phe Asp Val Asp Val Ser Gln Leu Pro Cys Gly Leu Asn Gly Ala Leu
   130 135 140
   Tyr Phe Val Ser Met Asp Ala Asp Gly Gly Val Ser Lys Tyr Pro Thr
   145 150 155 160
   Asn Thr Ala Gly Ala Lys Tyr Gly Thr Gly Tyr Cys Asp Ser Gln Cys
   165 170 175
   Pro Arg Asp Leu Lys Phe Ile Asn Gly Gln Ala Asn Val Glu Gly Trp
   180 185 190
   Glu Pro Ser Ser Asn Asn Ala Asn Thr Gly Ile Gly Gly His Gly Ser
   195 200 205
   Cys Cys Ser Glu Met Asp Ile Trp Glu Ala Asn Ser Ile Ser Glu Ala
   210 215 220
   Leu Thr Pro His Pro Cys Thr Thr Val Gly Gln Glu Ile Cys Glu Gly
   225 230 235 240
   Asp Gly Cys Gly Gly Thr Tyr Ser Asp Asn Arg Tyr Gly Gly Thr Cys
   245 250 255
   Asp Pro Asp Gly Cys Asp Trp Asn Pro Tyr Arg Leu Gly Asn Thr Ser
   260 265 270
   Phe Tyr Gly Pro Gly Ser Ser Phe Thr Leu Asp Thr Thr Lys Lys Leu
   275 280 285
   Thr Val Val Thr Gln Phe Glu Thr Ser Gly Ala Ile Asn Arg Tyr Tyr
   290 295 300
   Val Gln Asn Gly Val Thr Phe Gln Gln Pro Asn Ala Glu Leu Gly Ser
   305 310 315 320
   Tyr Ser Gly Asn Glu Leu Asn Asp Asp Tyr Cys Thr Ala Glu Glu Ala
   325 330 335
   Glu Phe Gly Gly Ser Ser Phe Ser Asp Lys Gly Gly Leu Thr Gln Phe
   340 345 350
   Lys Lys Ala Thr Ser Gly Gly Met Val Leu Val Met Ser Leu Trp Asp
   355 360 365
   Asp Tyr Tyr Ala Asn Met Leu Trp Leu Asp Ser Thr Tyr Pro Thr Asn
   370 375 380
   Glu Thr Ser Ser Thr Pro Gly Ala Val Arg Gly Ser Cys Ser Thr Ser
   385 390 395 400
   Ser Gly Val Pro Ala Gln Val Glu Ser Gln Ser Pro Asn Ala Lys Val
   405 410 415
   Thr Phe Ser Asn Ile Lys Phe Gly Pro Ile Gly Ser Thr Gly Asn Pro
   420 425 430
   Ser Gly Gly Asn Pro Pro Gly Gly Asn Arg Gly Thr Thr Thr Thr Arg
   435 440 445
   Arg Pro Ala Thr Thr Thr Gly Ser Ser Pro Gly Pro Thr Gln Ser His
   450 455 460
   Tyr Gly Gln Cys Gly Gly Ile Gly Tyr Ser Gly Pro Thr Val Cys Ala
   465 470 475 480
   Ser Gly Thr Thr Cys Gln Val Leu Asn Pro Tyr Tyr Ser Gln Cys Leu
   485 490 495
<210> 13
   <211> 1767
   <212> DNA
   <213> Artificial
<220>
   <223> coding sequence for Trichoderma viride CBHI, including the alpha factor signal peptide
<400> 13
   atgagatttc cttcaatttt tactgcagtt ttattcgcag catcctccgc attagctgct     60
   ccagtcaaca ctacaacaga agatgaaacg gcacaaattc cggctgaagc tgtcatcggt    120
   tacttagatt tagaagggga tttcgatgtt gctgttttgc cattttccaa cagcacaaat    180
   aacgggttat tgtttataaa tactactatt gccagcattg ctgctaaaga agaaggggta    240
   tctttggata aacgtgaggc ggaagcaccc tctcaatctg cttgcacctt gcagtctgaa    300
   actcacccac cattgacctg gcagaagtgt tcttctggcg gtacttgtac tcagcagacc    360
   ggttctgttg ttatcgacgc caactggaga tggactcacg ctaccaactc ttctaccaac    420
   tgctacgacg gtaacacttg gtcgtctacc ttgtgtccag acaacgagac ctgtgccaag    480
   aactgttgtt tggacggtgc tgcttacgct tctacctacg gtgttaccac ctctggtaac    540
   tcgctgtcta tcggtttcgt tacccagtct gcccagaaaa atgttggtgc cagactgtac    600
   ttgatggctt ctgacaccac ctaccaagag tttaccctgc tgggtaacga gttctctttc    660
   gacgtggacg tttctcaact gccatgtgga ctgaacggtg ccctgtactt cgtttctatg    720
   gacgctgacg gtggtgtttc taagtaccca accaacaccg ctggtgctaa atacggaacc    780
   ggttactgcg attctcagtg cccaagagac ctgaagttca tcaacggaca ggctaacgtt    840
   gaaggatggg agccatcttc taacaacgcc aacaccggta ttggtggtca cggttcttgc    900
   tgttctgaga tggacatctg ggaggccaac tctatttctg aggctttgac ccccacaccc    960
   tgtactactg tgggtcaaga gatctgtgag ggtgatggtt gtggtggtac ttactcggac   1020
   aacagatacg gtggtacttg tgacccagac ggttgtgatt gggacccata cagactgggt   1080
   aacacctctt tctacggtcc aggatcttct tttaccctgg acaccaccaa gaagttgacc   1140
   gttgttaccc agtttgagac ctctggtgcc atcaacagat actacgtgca gaacggtgtt   1200
   actttccagc agccaaacgc tgaactggga tcttactctg gtaacggact gaacgacgac   1260
   tactgtactg ctgaggaagc tgagttcggt ggttcttctt tctctgacaa gggtggactg   1320
   acccagttta agaaggctac ctctggcgga atggtgctgg ttatgtcttt gtgggacgac   1380
   tactacgcta acatgctgtg gcttgactct acctacccaa ctaacgagac ctcttctacc   1440
   ccaggtgctg ttagaggatc ttgctctacc tcttctggtg ttccagctca ggttgagtct   1500
   cagtctccaa acgccaaggt gaccttctct aacatcaagt tcggtccaat cggttctact   1560
   ggtgacccat ctggtggtaa cccaccaggt ggaaacccac ctggtactac cactaccaga   1620
   agaccagcta ccaccactgg ttcttctcca ggtccaaccc aatctcacta cggtcagtgt   1680
   ggtggtattg gttactctgg tccaaccgtt tgtgcttctg gaaccacctg tcaggttctg   1740
   aacccatact actcgcagtg cctgtaa                                       1767
<210> 14
   <211> 497
   <212> PRT
   <213> Artificial
<220>
   <223> Trichoderma viride CBHI (CBH-f)
<400> 14
   Gln Ser Ala Cys Thr Leu Gln Ser Glu Thr His Pro Pro Leu Thr Trp
   1               5                   10                  15
   Gln Lys Cys Ser Ser Gly Gly Thr Cys Thr Gln Gln Thr Gly Ser Val
               20                  25                  30
   Val Ile Asp Ala Asn Trp Arg Trp Thr His Ala Thr Asn Ser Ser Thr
           35                  40                  45
   Asn Cys Tyr Asp Gly Asn Thr Trp Ser Ser Thr Leu Cys Pro Asp Asn
       50                  55                  60
   Glu Thr Cys Ala Lys Asn Cys Cys Leu Asp Gly Ala Ala Tyr Ala Ser
   65                  70                  75                  80
   Thr Tyr Gly Val Thr Thr Ser Gly Asn Ser Leu Ser Ile Gly Phe Val
   85 90 95
   Thr Gln Ser Ala Gln Lys Asn Val Gly Ala Arg Leu Tyr Leu Met Ala
   100 105 110
   Ser Asp Thr Thr Tyr Gln Glu Phe Thr Leu Leu Gly Asn Glu Phe Ser
   115 120 125
   Phe Asp Val Asp Val Ser Gln Leu Pro Cys Gly Leu Asn Gly Ala Leu
   130 135 140
   Tyr Phe Val Ser Met Asp Ala Asp Gly Gly Val Ser Lys Tyr Pro Thr
   145 150 155 160
   Asn Thr Ala Gly Ala Lys Tyr Gly Thr Gly Tyr Cys Asp Ser Gln Cys
   165 170 175
   Pro Arg Asp Leu Lys Phe Ile Asn Gly Gln Ala Asn Val Glu Gly Trp
   180 185 190
   Glu Pro Ser Ser Asn Asn Ala Asn Thr Gly Ile Gly Gly His Gly Ser
   195 200 205
   Cys Cys Ser Glu Met Asp Ile Trp Glu Ala Asn Ser Ile Ser Glu Ala
   210 215 220
   Leu Thr Pro His Pro Cys Thr Thr Val Gly Gln Glu Ile Cys Glu Gly
   225 230 235 240
   Asp Gly Cys Gly Gly Thr Tyr Ser Asp Asn Arg Tyr Gly Gly Thr Cys
   245 250 255
   Asp Pro Asp Gly Cys Asp Trp Asp Pro Tyr Arg Leu Gly Asn Thr Ser
   260 265 270
   Phe Tyr Gly Pro Gly Ser Ser Phe Thr Leu Asp Thr Thr Lys Lys Leu
   275 280 285
   Thr Val Val Thr Gln Phe Glu Thr Ser Gly Ala Ile Asn Arg Tyr Tyr
   290 295 300
   Val Gln Asn Gly Val Thr Phe Gln Gln Pro Asn Ala Glu Leu Gly Ser
   305 310 315 320
   Tyr Ser Gly Asn Gly Leu Asn Asp Asp Tyr Cys Thr Ala Glu Glu Ala
   325 330 335
   Glu Phe Gly Gly Ser Ser Phe Ser Asp Lys Gly Gly Leu Thr Gln Phe
   340 345 350
   Lys Lys Ala Thr Ser Gly Gly Met Val Leu Val Met Ser Leu Trp Asp
   355 360 365
   Asp Tyr Tyr Ala Asn Met Leu Trp Leu Asp Ser Thr Tyr Pro Thr Asn
   370 375 380
   Glu Thr Ser Ser Thr Pro Gly Ala Val Arg Gly Ser Cys Ser Thr Ser
   385 390 395 400
   Ser Gly Val Pro Ala Gln Val Glu Ser Gln Ser Pro Asn Ala Lys Val
   405 410 415
   Thr Phe Ser Asn Ile Lys Phe Gly Pro Ile Gly Ser Thr Gly Asp Pro
   420 425 430
   Ser Gly Gly Asn Pro Pro Gly Gly Asn Pro Pro Gly Thr Thr Thr Thr
   435 440 445
   Arg Arg Pro Ala Thr Thr Thr Gly Ser Ser Pro Gly Pro Thr Gln Ser
   450 455 460
   His Tyr Gly Gln Cys Gly Gly Ile Gly Tyr Ser Gly Pro Thr Val Cys
   465 470 475 480
   Ala Ser Gly Thr Thr Cys Gln Val Leu Asn Pro Tyr Tyr Ser Gln Cys
   485 490 495
   Leu
<210> 15
   <211> 1785
   <212> DNA
   <213> Artificial
<220>
   <223> coding sequence for Humicola grisea CBHI- Trichoderma reesei CBHI cellulose binding domain fusion protein including the alpha factor signal peptide and a 6x His Tag
<400> 15
   atgagatttc cttcaatttt tactgcagtt ttattcgcag catcctccgc attagctgct 60
   ccagtcaaca ctacaacaga agatgaaacg gcacaaattc cggctgaagc tgtcatcggt 120
   tacttagatt tagaagggga tttcgatgtt gctgttttgc cattttccaa cagcacaaat 180
   aacgggttat tgtttataaa tactactatt gccagcattg ctgctaaaga agaaggggta 240
   tctttggata aacgtgaggc ggaagcatgc tcgcagcagg ctggtacaat tactgctgag 300
   aaccatccaa gaatgacgtg gaagagatgt agtggtccag gaaactgtca gactgttcag 360
   ggtgaggtcg tgatagatgc taactggaga tggttgcata acaacggcca gaactgctac 420
   gagggtaaca agtggacctc tcagtgttct tctgctaccg actgcgctca gagatgtgct 480
   cttgatggag caaactacca gagtacatat ggtgcttcta cctctggtga cagccttacc 540
   ctgaagtttg taaccaagca cgagtacgga accaatatcg gttctagatt ctacctgatg 600
   gctaaccaga acaagtacca gatgtttacc ttgatgaaca acgagttcgc cttcgacgta 660
   gatctgtcta aggtggagtg tggaatcaat tctgccttgt actttgtcgc tatggaagag 720
   gacggaggta tggcttctta cccttctaac agagctggtg ctaagtatgg aactggatac 780
   tgcgatgccc aatgcgctag agacctgaag ttcatcggtg gaaaggctaa cattgaaggt 840
   tggagacctt ctaccaacga cccaaacgct ggagttggtc caatgggtgc ttgctgtgcc 900
   gagattgacg tgtgggaatc taacgcttac gcctacgctt ttactccaca tgcttgcggt 960
   tctaagaaca gataccacat ttgcgaaacc aacaactgtg gtggcactta ctctgatgac 1020
   agattcgctg gatactgtga tgctaacgga tgtgattaca acccatacag aatgggtaac 1080
   aaggactttt acggaaaggg taagactgtt gacactaaca gaaagttcac tgtggtctcg 1140
   agatttgaga gaaacagact gtcgcagttc tttgtgcagg acggaagaaa gattgaggtc 1200
   ccaccaccaa cttggccagg attgccaaac tctgccgaca ttaccccaga gttgtgcgac 1260
   gctcagttca gagtgtttga cgacagaaac agatttgctg agaccggtgg atttgacgct 1320
   ttgaacgagg ctctgaccat tccaatggtt ctagtcatga gtatttggga cgatcaccac 1380
   tctaacatgc tttggctgga ctcttcttac cctccagaga aggctggatt gcctggtggt 1440
   gacagaggtc catgtccaac aacttctgga gttccagccg aggttgaggc tcaataccca 1500
   gacgcccagg tcgtgtggtc caacatcaga ttcggaccaa ttggaagctt aacaggtaat 1560
   ccttcaggtg gtaatcctcc aggtggaaac agaggaacaa cgacaactag aagaccagct 1620
   actacaactg gttcaagtcc aggtccaact caatcacact acggtcaatg tggtggtata 1680
   ggttactctg gtcccactgt ttgtgcttct ggtactactt gccaagttct gaacccttac 1740
   tactcacagt gtctagcttc tgcacaccat catcatcatc attaa 1785
<210> 16
   <211> 503
   <212> PRT
   <213> Artificial
<220>
   <223> Humicola grisea CBHI- Trichoderma reesei CBHI cellulose binding domain fusion protein including a 6x His Tag (CBH-g)
<400> 16
   Gln Gln Ala Gly Thr Ile Thr Ala Glu Asn His Pro Arg Met Thr Trp
   1               5                   10                  15
   Lys Arg Cys Ser Gly Pro Gly Asn Cys Gln Thr Val Gln Gly Glu Val
               20                  25                  30
   Val Ile Asp Ala Asn Trp Arg Trp Leu His Asn Asn Gly Gln Asn Cys
           35                  40                  45
   Tyr Glu Gly Asn Lys Trp Thr Ser Gln Cys Ser Ser Ala Thr Asp Cys
       50                  55                  60
   Ala Gln Arg Cys Ala Leu Asp Gly Ala Asn Tyr Gln Ser Thr Tyr Gly
   65                  70                  75                  80
   Ala Ser Thr Ser Gly Asp Ser Leu Thr Leu Lys Phe Val Thr Lys His
                   85                  90                  95
   Glu Tyr Gly Thr Asn Ile Gly Ser Arg Phe Tyr Leu Met Ala Asn Gln
               100                 105                 110
   Asn Lys Tyr Gln Met Phe Thr Leu Met Asn Asn Glu Phe Ala Phe Asp
           115                 120                 125
   Val Asp Leu Ser Lys Val Glu Cys Gly Ile Asn Ser Ala Leu Tyr Phe
       130                 135                 140
   Val Ala Met Glu Glu Asp Gly Gly Met Ala Ser Tyr Pro Ser Asn Arg
   145                 150                 155                 160
   Ala Gly Ala Lys Tyr Gly Thr Gly Tyr Cys Asp Ala Gln Cys Ala Arg
                   165                 170                 175
   Asp Leu Lys Phe Ile Gly Gly Lys Ala Asn Ile Glu Gly Trp Arg Pro
               180                 185                 190
   Ser Thr Asn Asp Pro Asn Ala Gly Val Gly Pro Met Gly Ala Cys Cys
           195                 200                 205
   Ala Glu Ile Asp Val Trp Glu Ser Asn Ala Tyr Ala Tyr Ala Phe Thr
       210                 215                 220
   Pro His Ala Cys Gly Ser Lys Asn Arg Tyr His Ile Cys Glu Thr Asn
   225                 230                 235                 240
   Asn Cys Gly Gly Thr Tyr Ser Asp Asp Arg Phe Ala Gly Tyr Cys Asp
                   245                 250                 255
   Ala Asn Gly Cys Asp Tyr Asn Pro Tyr Arg Met Gly Asn Lys Asp Phe
               260                 265                 270
   Tyr Gly Lys Gly Lys Thr Val Asp Thr Asn Arg Lys Phe Thr Val Val
           275                 280                 285
   Ser Arg Phe Glu Arg Asn Arg Leu Ser Gln Phe Phe Val Gln Asp Gly
       290                 295                 300
   Arg Lys Ile Glu Val Pro Pro Pro Thr Trp Pro Gly Leu Pro Asn Ser
   305                 310                 315                 320
   Ala Asp Ile Thr Pro Glu Leu Cys Asp Ala Gln Phe Arg Val Phe Asp
                   325                 330                 335
   Asp Arg Asn Arg Phe Ala Glu Thr Gly Gly Phe Asp Ala Leu Asn Glu
   340 345 350
   Ala Leu Thr Ile Pro Met Val Leu Val Met Ser Ile Trp Asp Asp His
   355 360 365
   His Ser Asn Met Leu Trp Leu Asp Ser Ser Tyr Pro Pro Glu Lys Ala
   370 375 380
   Gly Leu Pro Gly Gly Asp Arg Gly Pro Cys Pro Thr Thr Ser Gly Val
   385 390 395 400
   Pro Ala Glu Val Glu Ala Gln Tyr Pro Asp Ala Gln Val Val Trp Ser
   405 410 415
   Asn Ile Arg Phe Gly Pro Ile Gly Ser Leu Thr Gly Asn Pro Ser Gly
   420 425 430
   Gly Asn Pro Pro Gly Gly Asn Arg Gly Thr Thr Thr Thr Arg Arg Pro
   435 440 445
   Ala Thr Thr Thr Gly Ser Ser Pro Gly Pro Thr Gln Ser His Tyr Gly
   450 455 460
   Gln Cys Gly Gly Ile Gly Tyr Ser Gly Pro Thr Val Cys Ala Ser Gly
   465 470 475 480
   Thr Thr Cys Gln Val Leu Asn Pro Tyr Tyr Ser Gln Cys Leu Ala Ser
   485 490 495
   Ala His His His His His His
   500
<210> 17
   <211> 1809
   <212> DNA
   <213> Artificial
<220>
   <223> Coding sequence for Talaromyces emersonii CBHI / Trichoderma reesei -CBD fusion including the alpha factor signal peptide and a 6x His Tag
<400> 17
   atgagatttc cttcaatttt tactgcagtt ttattcgcag catcctccgc attagctgct 60
   ccagtcaaca ctacaacaga agatgaaacg gcacaaattc cggctgaagc tgtcatcggt 120
   tacttagatt tagaagggga tttcgatgtt gctgttttgc cattttccaa cagcacaaat 180
   aacgggttat tgtttataaa tactactatt gccagcattg ctgctaaaga agaaggggta 240
   tctttggata aacgtgaggc ggaagcatgc tcgcagcagg ccggcacggc gacggcagag 300
   aaccacccgc ccctgacatg gcaggaatgc accgcccctg ggagctgcac cacccagaac 360
   ggggcggtcg ttcttgatgc gaactggcgt tgggtgcacg atgtgaacgg atacaccaac 420
   tgctacacgg gcaatacctg ggaccccacg tactgccctg acgacgaaac ctgcgcccag 480
   aactgtgcgc tggacggcgc ggattacgag ggcacctacg gcgtgacttc gtcgggcagc 540
   tccttgaaac tcaatttcgt caccgggtcg aacgtcggat cccgtctcta cctgctgcag 600
   gacgactcga cctatcagat cttcaagctc ctgaaccgcg agttcagctt tgacgtcgat 660
   gtctccaatc ttccgtgcgg attgaacggc gctctgtact ttgtcgccat ggacgccgac 720
   ggcggcgtgt ccaagtaccc gaacaacaag gctggtgcca agtacggaac cgggtattgc 780
   gactcccaat gcccacggga cctcaagttc atcgacggcg aggccaacgt cgagggctgg 840
   cagccgtctt cgaacaacgc caacaccgga attggcgacc acggctcctg ctgtgcggag 900
   atggatgtct gggaagcaaa cagcatctcc aatgcggtca ctccgcaccc gtgcgacacg 960
   ccaggccaga cgatgtgctc tggagatgac tgcggtggca catactctaa cgatcgctac 1020
   gcgggaacct gcgatcctga cggctgtgac ttcaacccct accgcatggg caacacttct 1080
   ttctacgggc ctggcaagat catcgatacc accaagccct tcactgtcgt gacgcagttc 1140
   ctcactgatg atggtacgga tactggaact ctcagcgaga tcaagcgctt ctacatccag 1200
   aacagcaacg tcattccgca gcccaactcg gacatcagtg gcgtgaccgg caactcgatc 1260
   acgacggagt tctgcactgc tcagaagcag gcctttggcg acacggacga cttctctcag 1320
   cacggtggcc tggccaagat gggagcggcc atgcagcagg gtatggtcct ggtgatgagt 1380
   ttgtgggacg actacgccgc gcagatgctg tggttggatt ccgactaccc gacggatgcg 1440
   gaccccacga cccctggtat tgcccgtgga acgtgtccga cggactcggg cgtcccatcg 1500
   gatgtcgagt cgcagagccc caactcctac gtgacctact cgaacattaa gtttggtccg 1560
   atcggtagca caggtaatcc ttcaggtggt aatcctccag gtggaaacag aggaacaacg 1620
   acaactagaa gaccagctac tacaactggt tcaagtccag gtccaactca atcacactac 1680
   ggtcaatgtg gtggtatagg ttactctggt cccactgttt gtgcttctgg tactacttgc 1740
   caagttctga acccttacta ctcacagtgt ctagcttctg cacatcatca ccaccaccat 1800
   taatgataa 1809
<210> 18
   <211> 509
   <212> PRT
   <213> Artificial
<220>
   <223> Mature Sequence of Talaromyces emersonii CBHI / Trichoderma reesei -CBD fusion with 6x-His tag (CBH-ah)
<400> 18
   Gln Gln Ala Gly Thr Ala Thr Ala Glu Asn His Pro Pro Leu Thr Trp
   1               5                   10                  15
   Gln Glu Cys Thr Ala Pro Gly Ser Cys Thr Thr Gln Asn Gly Ala Val
               20                  25                  30
   Val Leu Asp Ala Asn Trp Arg Trp Val His Asp Val Asn Gly Tyr Thr
           35                  40                  45
   Asn Cys Tyr Thr Gly Asn Thr Trp Asp Pro Thr Tyr Cys Pro Asp Asp
       50                  55                  60
   Glu Thr Cys Ala Gln Asn Cys Ala Leu Asp Gly Ala Asp Tyr Glu Gly
   65                  70                  75                  80
   Thr Tyr Gly Val Thr Ser Ser Gly Ser Ser Leu Lys Leu Asn Phe Val
                   85                  90                  95
   Thr Gly Ser Asn Val Gly Ser Arg Leu Tyr Leu Leu Gln Asp Asp Ser
               100                 105                 110
   Thr Tyr Gln Ile Phe Lys Leu Leu Asn Arg Glu Phe Ser Phe Asp Val
           115                 120                 125
   Asp Val Ser Asn Leu Pro Cys Gly Leu Asn Gly Ala Leu Tyr Phe Val
       130                 135                 140
   Ala Met Asp Ala Asp Gly Gly Val Ser Lys Tyr Pro Asn Asn Lys Ala
   145                 150                 155                 160
   Gly Ala Lys Tyr Gly Thr Gly Tyr Cys Asp Ser Gln Cys Pro Arg Asp
                   165                 170                 175
   Leu Lys Phe Ile Asp Gly Glu Ala Asn Val Glu Gly Trp Gln Pro Ser
               180                 185                 190
   Ser Asn Asn Ala Asn Thr Gly Ile Gly Asp His Gly Ser Cys Cys Ala
           195                 200                 205
   Glu Met Asp Val Trp Glu Ala Asn Ser Ile Ser Asn Ala Val Thr Pro
       210                 215                 220
   His Pro Cys Asp Thr Pro Gly Gln Thr Met Cys Ser Gly Asp Asp Cys
   225                 230                 235                 240
   Gly Gly Thr Tyr Ser Asn Asp Arg Tyr Ala Gly Thr Cys Asp Pro Asp
                   245                 250                 255
   Gly Cys Asp Phe Asn Pro Tyr Arg Met Gly Asn Thr Ser Phe Tyr Gly
               260                 265                 270
   Pro Gly Lys Ile Ile Asp Thr Thr Lys Pro Phe Thr Val Val Thr Gln
           275                 280                 285
   Phe Leu Thr Asp Asp Gly Thr Asp Thr Gly Thr Leu Ser Glu Ile Lys
       290                 295                 300
   Arg Phe Tyr Ile Gln Asn Ser Asn Val Ile Pro Gln Pro Asn Ser Asp
   305                 310                 315                 320
   Ile Ser Gly Val Thr Gly Asn Ser Ile Thr Thr Glu Phe Cys Thr Ala
   325 330 335
   Gln Lys Gln Ala Phe Gly Asp Thr Asp Asp Phe Ser Gln His Gly Gly
   340 345 350
   Leu Ala Lys Met Gly Ala Ala Met Gln Gln Gly Met Val Leu Val Met
   355 360 365
   Ser Leu Trp Asp Asp Tyr Ala Ala Gln Met Leu Trp Leu Asp Ser Asp
   370 375 380
   Tyr Pro Thr Asp Ala Asp Pro Thr Thr Pro Gly Ile Ala Arg Gly Thr
   385 390 395 400
   Cys Pro Thr Asp Ser Gly Val Pro Ser Asp Val Glu Ser Gln Ser Pro
   405 410 415
   Asn Ser Tyr Val Thr Tyr Ser Asn Ile Lys Phe Gly Pro Ile Gly Ser
   420 425 430
   Thr Gly Asn Pro Ser Gly Gly Asn Pro Pro Gly Gly Asn Arg Gly Thr
   435 440 445
   Thr Thr Thr Arg Arg Pro Ala Thr Thr Thr Gly Ser Ser Pro Gly Pro
   450 455 460
   Thr Gln Ser His Tyr Gly Gln Cys Gly Gly Ile Gly Tyr Ser Gly Pro
   465 470 475 480
   Thr Val Cys Ala Ser Gly Thr Thr Cys Gln Val Leu Asn Pro Tyr Tyr
   485 490 495
   Ser Gln Cys Leu Ala Ser Ala His His His His His His
   500 505
<210> 19
   <211> 1335
   <212> DNA
   <213> Artificial
<220>
   <223> Alternative coding sequence of Humicola grisea CBHI with signal sequence
<400> 19
   atggccagcg atctggcaca gcaggctggt acaattactg ctgagaacca tccaagaatg 60
   acgtggaaga gatgtagtgg tccaggaaac tgtcagactg ttcagggtga ggtcgtgata 120
   gatgctaact ggagatggtt gcataacaac ggccagaact gctacgaggg taacaagtgg 180
   acctctcagt gttcttctgc taccgactgc gctcagagat gtgctcttga tggagcaaac 240
   taccagagta catatggtgc ttctacctct ggtgacagcc ttaccctgaa gtttgtaacc 300
   aagcacgagt acggaaccaa tatcggttct agattctacc tgatggctaa ccagaacaag 360
   taccagatgt ttaccttgat gaacaacgag ttcgccttcg acgtagatct gtctaaggtg 420
   gagtgtggaa tcaattctgc cttgtacttt gtcgctatgg aagaggacgg aggtatggct 480
   tcttaccctt ctaacagagc tggtgctaag tatggaactg gatactgcga tgcccaatgc 540
   gctagagacc tgaagttcat cggtggaaag gctaacattg aaggttggag accttctacc 600
   aacgacccaa acgctggagt tggtccaatg ggtgcttgct gtgccgagat tgacgtgtgg 660
   gaatctaacg cttacgccta cgcttttact ccacatgctt gcggttctaa gaacagatac 720
   cacatttgcg aaaccaacaa ctgtggtggc acttactctg atgacagatt cgctggatac 780
   tgtgatgcta acggatgtga ttacaaccca tacagaatgg gtaacaagga cttttacgga 840
   aagggtaaga ctgttgacac taacagaaag ttcactgtgg tctcgagatt tgagagaaac 900
   agactgtcgc agttctttgt gcaggacgga agaaagattg aggtcccacc accaacttgg 960
   ccaggattgc caaactctgc cgacattacc ccagagttgt gcgacgctca gttcagagtg 1020
   tttgacgaca gaaacagatt tgctgagacc ggtggatttg acgctttgaa cgaggctctg 1080
   accattccaa tggttctagt catgagtatt tgggacgatc accactctaa catgctttgg 1140
   ctggactctt cttaccctcc agagaaggct ggattgcctg gtggtgacag aggtccatgt 1200
   ccaacaactt ctggagttcc agccgaggtt gaggctcaat acccagacgc ccaggtcgtg 1260
   tggtccaaca tcagattcgg accaattggt agcacagtga atgtggcttc tgcacaccat 1320
   catcatcatc attga 1335
<210> 20
   <211> 41
   <212> DNA
   <213> Artificial
<220>
   <223> Primer forward
<400> 20
   gaggcggaag caccctctca atctgcttgc accttgcagt c 41
<210> 21
   <211> 38
   <212> DNA
   <213> Artificial
<220>
   <223> Primer reverse
<400> 21
   ggagacgcag agcccttatt acaggcactg cgagtagt 38
<210> 22
   <211> 41
   <212> DNA
   <213> Artificial
<220>
   <223> Primer forward
<400> 22
   gaggcggaag caccctctca gcaggctggt actattactg c 41
<210> 23
   <211> 44
   <212> DNA
   <213> Artificial
<220>
   <223> Primer reverse
<400> 23
   ggagacgcag agcccttaca cgttcacggt agaaccgatt gggc 44
<210> 24
   <211> 41
   <212> DNA
   <213> Artificial
<220>
   <223> Primer forward
<400> 24
   gaggcggaag caccctctca cgaggccggt accgtaaccg c 41
<210> 25
   <211> 41
   <212> DNA
   <213> Artificial
<220>
   <223> Primer reverse
<400> 25
   ggagacgcag agcccttatt agttggcggt gaaggtcgag t 41
<210> 26
   <211> 41
   <212> DNA
   <213> Artificial
<220>
   <223> Primer forward
<400> 26
   gaggcggaag caccctctca gcaggccggc acggcgacgg c 41
<210> 27
   <211> 41
   <212> DNA
   <213> Artificial
<220>
   <223> Primer reverse
<400> 27
   ggagacgcag agcccttatc acgaagcggt gaaggtcgag t 41
<210> 28
   <211> 41
   <212> DNA
   <213> Artificial
<220>
   <223> Primer forward
<400> 28
   gaggcggaag caccctctca gcaggccggc acggcgacgg c 41
<210> 29
   <211> 38
   <212> DNA
   <213> Artificial
<220>
   <223> Primer reverse
<400> 29
   attacctgtg ctaccgatcg gaccaaactt aatgttcg 38
<210> 30
   <211> 38
   <212> DNA
   <213> Artificial
<220>
   <223> Primer forward
<400> 30
   aagtttggtc cgatcggtag cacaggtaat ccttcagg 38
<210> 31
   <211> 44
   <212> DNA
   <213> Artificial
<220>
   <223> Primer reverse
<400> 31
   ggagacgcag agcccttatt atagacactg tgagtagtaa gggt 44
<210> 32
   <211> 41
   <212> DNA
   <213> Artificial
<220>
   <223> Primer forward
<400> 32
   gaggcggaag caccctctca gcaggccggc acggcgacggc 41
<210> 33
   <211> 44
   <212> DNA
   <213> Artificial
<220>
   <223> Primer reverse
<400> 33
   ggagacgcag agcccttatc attaatggtg gtggtgatga tgag 44
<210> 34
   <211> 40
   <212> DNA
   <213> Artificial
<220>
   <223> Primer forward
<400> 34
   aggcggaagc atgctcgcag caggctggta caattactgc 40
<210> 35
   <211> 41
   <212> DNA
   <213> Artificial
<220>
   <223> Primer reverse
<400> 35
   ggattacctg ttaagcttcc aattggtccg aatctgatgt t 41
<210> 36
   <211> 42
   <212> DNA
   <213> Artificial
<220>
   <223> Primer forward
<400> 36
   accaattgga agcttaacag gtaatccttc aggtggtaat cc 42
<210> 37
   <211> 46
   <212> DNA
   <213> Artificial
<220>
   <223> Primer reverse
<400> 37
   atcttgcagg tcgacttatc attaatgatg atgatgatgg tgtgca 46
<210> 38
   <211> 40
   <212> DNA
   <213> Artificial
<220>
   <223> Primer forward
<400> 38
   aggcggaagc atgctcgcag caggctggta caattactgc 40
<210> 39
   <211> 46
   <212> DNA
   <213> Artificial
<220>
   <223> Primer reverse
<400> 39
   atcttgcagg tcgacttatc attaatgatg atgatgatgg tgtgca 46
<210> 40
   <211> 21
   <212> DNA
   <213> Artificial
<220>
   <223> oligonucleotide alpha-f
<400> 40
   tactattgcc agcattgctg c 21
<210> 41
   <211> 23
   <212> DNA
   <213> Artificial
<220>
   <223> Oligonucleotide oli740
<400> 41
   tcagctattt cacatacaaa tcg 23